# EUROPEAN PATENT APPLICATION

(11) **EP 4 647 425 A1**
(43) Date of publication of application: **12.11.2025**
(21) Application number: 24899083.0
(22) Date of filing: 13.03.2024
(51) Int. Cl.: C07D 249/18, H10F 19/80, C09J 7/30

(54) **BENZOTRIAZOLE ORGANIC COMPOUND, AND PREPARATION METHOD THEREFOR AND USE THEREOF**

(30) Priority: 08.12.2023 CN 202311682870
(71) Applicant: Trina Solar Co., Ltd., Changzhou, Jiangsu 213031 (CN)
(72) Inventor: XU, Ye, Changzhou, Jiangsu 213031 (CN); CUI, Biao, Changzhou, Jiangsu 213031 (CN)
(74) Representative: Arnold & Siedsma
(86) International application number: PCT/CN2024/081440
(87) International publication number: WO 2025/118428

(57) **Abstract**

A benzotriazole organic compound, a preparation method thereof, and use thereof are provided. The benzotriazole organic compound has a structure represented by formula (1): wherein each Ar independently includes a substituted or unsubstituted aryl, or a substituted or unsubstituted heteroaryl; R₁ and R₂ are each independently selected from a substituted or unsubstituted alkyl group, or a substituted or unsubstituted alkoxy group; R₃ and R₄ are each independently selected from a halogen, a substituted or unsubstituted alkoxy group, or a substituted or unsubstituted alkylthio group; and R₅ is selected from a substituted or unsubstituted alkyl group.

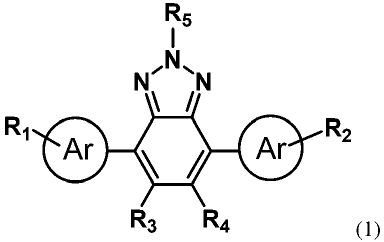

## Description

### RELATED APPLICATIONS

This application claims priority to Chinese patent application No. 2023116828707, entitled "BENZOTRIAZOLE ORGANIC COMPOUND, AND PREPARATION METHOD THEREFOR AND USE THEREOF" filed on December 08, 2023, the content of which is hereby incorporated by reference in its entirety.

### TECHNICAL FIELD

This application relates to the field of organic functional materials, in particular to a benzotriazole organic compound, a preparation method thereof, and use thereof.

### BACKGROUND

Solar energy, as a clean energy source that is abundant and requires no transportation, has the potential to become an alternative energy to conventional fossil fuels. The development of solar energy harvesting and conversion technologies has gradually become a research focus. Photovoltaic devices, which can directly convert solar energy into electricity, are one of the most efficient ways to utilize solar energy. To date, the global demand for photovoltaic device installations continues to rise.

However, the vast majority of photovoltaic devices can only efficiently utilize visible light and near-infrared light from sunlight, while the utilization efficiency for ultraviolet light with a wavelength less than 400 nm is low. Moreover, the presence of ultraviolet light in sunlight can reduce the service life of photovoltaic devices, especially for heterojunction (HJT) solar cells. HJT solar cells are expected to replace passivated emitter rear contact (PERC) cells and tunnel oxide passivated contact (TOPCon) cells, becoming the third-generation solar cells. In addition, the stability of photovoltaic devices can be improved by using encapsulating films with ultraviolet light absorption capabilities, but the efficiency of photovoltaic devices decreases seriously.

The use of wavelength conversion films not only allows effective absorption of ultraviolet light from sunlight to prevent damage to the service life of photovoltaic devices, but also converts ultraviolet light into usable visible light, thereby improving the photoelectric conversion efficiency of photovoltaic devices. The core technology of wavelength conversion films heavily lies in the selection of suitable light conversion agents. Conventional light conversion agents are mainly divided into inorganic light conversion agents and organic light conversion agents. Compared to the inorganic light conversion agents, the organic light conversion agents offer advantages such as lower price, greater variety, and higher tunability of performance. Additionally, since the substrates of the wavelength conversion films are typically organic polymer materials, the organic light conversion agents generally exhibit better dispersibility and compatibility with the substrates, resulting in less impact on the light transmittance of the wavelength conversion films. Although conventional organic light conversion agents can effectively absorb ultraviolet light and convert it into visible light (typically blue or sky-blue light), thereby improving the efficiency of photovoltaic devices, the stability of the conventional organic light conversion agents is poor, and the corresponding wavelength conversion films suffer from high water-vapor transmission rates, significantly reducing the service life of photovoltaic devices.

### SUMMARY

According to various embodiments of the present application, the present application provides a benzotriazole organic compound, a preparation method thereof, and use thereof.

The technical solutions are as follows:

In a first aspect of the present application, a benzotriazole organic compound is provided, having a general structural formula (1): 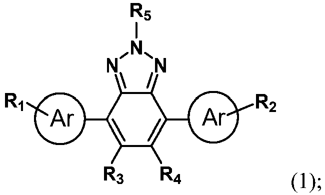
wherein each Ar independently includes substituted or unsubstituted aryl, or substituted or unsubstituted heteroaryl;
R₁ and R₂ are each independently selected from substituted or unsubstituted alkyl, or substituted or unsubstituted alkoxy;
R₃ and R₄ are each independently selected from halogen, substituted or unsubstituted alkoxy, or substituted or unsubstituted alkylthio; and
R₅ is selected from substituted or unsubstituted alkyl.

In some embodiments, each Ar independently includes substituted or unsubstituted aryl having 6 to 30 ring atoms, or substituted or unsubstituted heteroaryl having 5 to 30 ring atoms.

In some embodiments, each Ar is independently selected from substituted or unsubstituted six-membered aryl, substituted or unsubstituted six-membered heteroaryl, or substituted or unsubstituted five-membered heteroaryl.

In some embodiments, each Ar is independently selected from one of following groups: 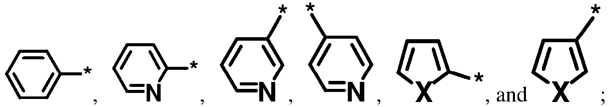 wherein * represents a linking site, and X represents O, S, or Se.

In some embodiments, R₁ and R₂ are each independently selected from substituted or unsubstituted C₁ to C₂₀ alkyl, or substituted or unsubstituted C₁ to C₂₀ alkoxy.

In some embodiments, R₁ and R₂ are each independently selected from one of following groups: 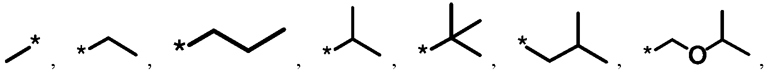 and 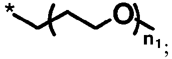 wherein * represents a linking site, and n₁≥2.

In some embodiments, R₃ and R₄ are each independently selected from halogen, substituted or unsubstituted C₁ to C₂₀ alkoxy, or substituted or unsubstituted C₁ to C₂₀ alkylthio.

In some embodiments, R₃ and R₄ are each independently selected from one of following groups: 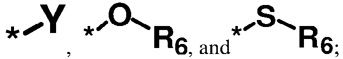 wherein * represents a linking site, Y represents F, Cl, Br, or I, and each R₆ is independently selected from substituted or unsubstituted C₁ to C₂₀ linear alkyl, or substituted or unsubstituted C₃ to C₂₀ branched alkyl.

In some embodiments, R₃ and R₄ are each independently selected from one of following groups: 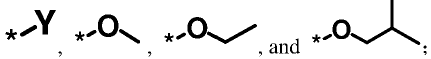 wherein * represents a linking site, Y represents F, Cl, Br, or I.

In some embodiments, R₅ is selected from substituted or unsubstituted C₁ to C₂₀ alkyl.

In some embodiments, R₅ is selected from one of following groups: 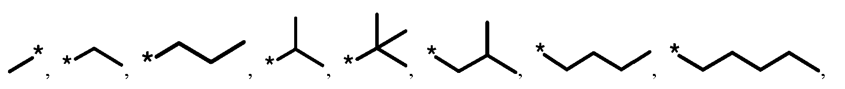 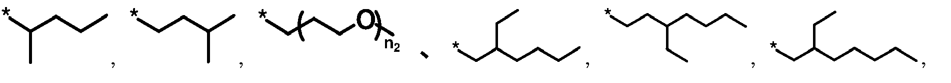 and 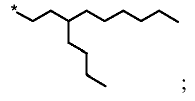 wherein * represents a linking site, and n₂≥2.

In some embodiments, the benzotriazole organic compound as described above has any one of the following structures:

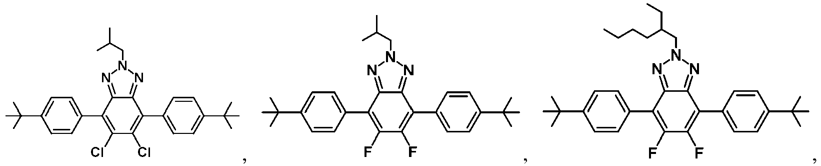

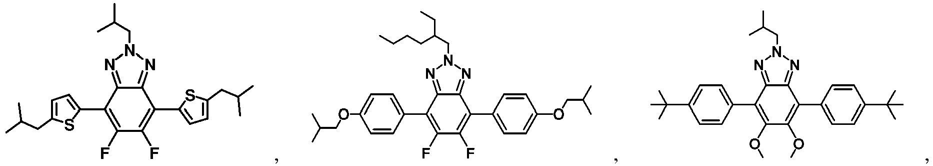

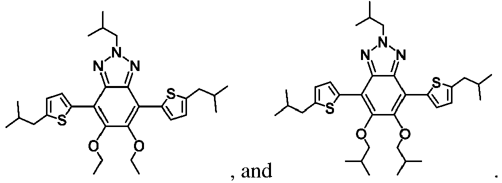

A second aspect of the present application further provides a method for preparing the benzotriazole organic compound as described above. The technical solutions are as follows:

A method for preparing the benzotriazole organic compound as described above includes the following steps:
mixing and reacting compound (a), compound (b), compound (c), and an alkali in a solvent to prepare the benzotriazole organic compound represented by formula (1);

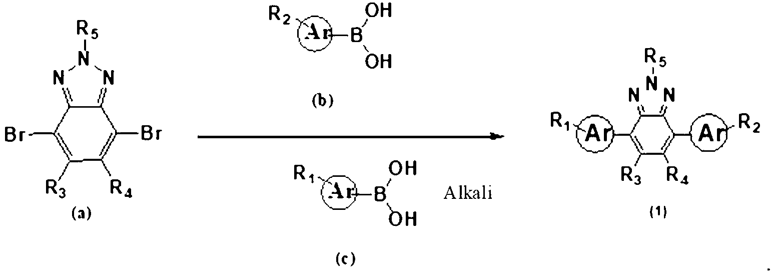

In some embodiments, a molar ratio of compound (a), compound (b), and compound (c) is substantially 1:(1 to 2):(1 to 2).

In some embodiments, a molar ratio of the alkali to compound (a) is substantially (1 to 2):1.

In some embodiments, the alkali includes potassium carbonate.

In some embodiments, the solvent includes a mixed solvent of 1,4-dioxane and water.

In some embodiments, the reaction temperature is in a range from about 80 °C to about 100 °C, and the reaction time period is in a range from about 20 hours to about 40 hours.

A third aspect of the present application further provides use of the benzotriazole organic compound as described above. The technical solutions are as follows:

A light conversion agent includes the benzotriazole organic compound as described above.

In some embodiments, the light conversion agent has a light absorption in a wavelength range from about 280 nm to about 400 nm.

In some embodiments, the light conversion agent has a maximum absorption peak at about 320 nm to about 380 nm.

In some embodiments, the light conversion agent has a photoluminescence in a range from about 400 nm to about 600 nm.

In some embodiments, the light conversion agent has a maximum emission peak at about 450 nm to about 550 nm.

A photovoltaic encapsulant film includes the benzotriazole organic compound as described above, or includes the light conversion agent as described above.

In some embodiments, the photovoltaic encapsulant film further includes an optically transparent polymer material.

In some embodiments, raw materials for preparing the photovoltaic encapsulant film includes, by mass percentage, about 0.01% to about 5% of the benzotriazole organic compound as described above or the light conversion agent as described above, and about 95% to about 99.99% of the polymer material.

In some embodiments, the polymer material is one or more selected from the group consisting of a polyolefin elastomer (POE), an ethylene-vinyl acetate copolymer (EVA), a polyvinyl butyral (PVB), and an organic silicone resin material.

In some embodiments, the organic silicone resin material includes a mixture of a hydrogen-containing silicone and a vinyl-terminated polydimethylsiloxane in a mass ratio of substantially 1:(2 to 15).

A photovoltaic device includes a solar cell and the photovoltaic encapsulant film as described above, wherein the photovoltaic encapsulant film is disposed on at least one surface of the solar cell.

In some embodiments, the solar cell includes a crystalline silicon cell, a perovskite cell, a tandem cell including the perovskite cell and the crystalline silicon cell, or any combination thereof.

In some embodiments, the solar cell is the tandem cell including the perovskite cell and the crystalline silicon cell, and the solar cell includes a first transparent conductive layer, an electron transport layer, a perovskite active layer, a hole transport layer, a second transparent conductive layer, an N-type doped layer, a first amorphous silicon layer, a silicon wafer, a second amorphous silicon layer, a P-type doped layer, and a third transparent conductive layer stacked in sequence, and further includes a first finger electrode layer and a second finger electrode layer,
wherein the first finger electrode layer includes a plurality of first finger electrodes spaced apart from each other, and each first finger electrode is electrically connected to the first transparent conductive layer; and
the second finger electrode layer includes a plurality of second finger electrodes spaced apart from each other, and each second finger electrode is electrically connected to the third transparent conductive layer.

In some embodiments, the photovoltaic device further includes a light-receiving surface plate and a back surface plate, the light-receiving surface plate is stacked on a surface of the first transparent conductive layer away from the perovskite active layer, the back surface plate is stacked on a surface of the third transparent conductive layer away from the perovskite active layer, and surfaces of the solar cell are coated with the photovoltaic encapsulant films.

In some embodiments, the solar cell further includes a first anti-reflection layer, the first anti-reflection layer is stacked on the surface of the first transparent conductive layer away from the perovskite active layer, and the first finger electrodes extend through the first anti-reflection layer and are electrically connected to the first transparent conductive layer;
a material of the first anti-reflection layer includes magnesium fluoride, lithium fluoride, silicon nitride, silicon oxide, silicon oxynitride, or any combination thereof.

In some embodiments, the solar cell further includes a second anti-reflection layer, the second anti-reflection layer is stacked on the surface of the first transparent conductive layer away from the perovskite active layer, the first finger electrodes extend through the second anti-reflection layer and are electrically connected to the first transparent conductive layer;
the second anti-reflection layer includes a patterned photovoltaic encapsulant film;
the photovoltaic encapsulant film includes, by mass percentage, about 0.01% to about 5% of the benzotriazole organic compound as described above or the light conversion agent as described above, and about 95% to about 99.99% of the polymer material; and
the organic silicone resin material includes a mixture of a hydrogen-containing silicone and a vinyl-terminated polydimethylsiloxane in a mass ratio of substantially 1:(2 to 15).

In some embodiment, the solar cell further includes a third anti-reflection layer, the third anti-reflection layer is stacked on the surface of the third transparent conductive layer away from the perovskite active layer, the second finger electrodes extend through the third anti-reflection layer and are electrically connected to the third transparent conductive layer;
the third anti-reflection layer includes a patterned photovoltaic encapsulant film;
the photovoltaic encapsulant film includes, by mass percentage, about 0.01% to about 5% of the benzotriazole organic compound as described above or the light conversion agent as described above, and about 95% to about 99.99% of the polymer material.
the organic silicone resin material includes a mixture of a hydrogen-containing silicone and a vinyl-terminated polydimethylsiloxane in a mass ratio of substantially 1:(2 to 15).

Details of one or more embodiments of the present application are set forth in the accompanying drawings and description below. Other features, objectives, and advantages of the present application will become apparent from the description, drawings, and claims.

### BRIEF DESCRIPTION OF THE DRAWINGS

To illustrate the technical solutions in the embodiments of the present application or in related art more clearly, the drawings used in the embodiments or in the related art will be described briefly. Apparently, the following described drawings are merely for the embodiments of the present application, and other drawings can be derived by those of ordinary skill in the art according to the disclosed drawings without any creative effort.
FIG. 1 is a schematic structural view of a tandem cell including a perovskite cell and a crystalline silicon cell according to an embodiment of the present application.
FIG. 2 is a schematic structural view of a tandem cell including a perovskite cell and a crystalline silicon cell according to another embodiment of the present application.
FIG. 3 is a schematic structural view of a tandem cell including a perovskite cell and a crystalline silicon cell according to yet another embodiment of the present application.
FIG. 4 is a schematic structural view of a photovoltaic device according to an embodiment of the present application.
FIG. 5 is a schematic structural view of a solar cell containing a single patterned anti-reflection layer according to an embodiment of the present application.
FIG. 6 is a schematic structural view of a solar cell including two patterned anti-reflection layers according to an embodiment of the present application.
FIG. 7 shows a flowchart of a method for preparing the solar cell containing two patterned anti-reflection layers as shown in FIG. 6, and a schematic structural view of intermediate structures thereof.
FIG. 8 is a schematic structural view of photovoltaic encapsulant films with pyramid-shaped and hemispherical patterns.
FIG. 9 shows a proton nuclear magnetic resonance (¹H NMR) spectrum of a benzotriazole compound synthesized in Synthesis Example 1 of the present application.
FIG. 10 shows a ¹H NMR spectrum of a benzotriazole compound synthesized in Synthesis Example 2 of the present application.
FIG. 11 shows a ¹H NMR spectrum of a benzotriazole compound synthesized in Synthesis Example 3 of the present application.
FIG. 12 shows a ¹H NMR spectrum of a benzotriazole compound synthesized in Synthesis Example 4 of the present application.
FIG. 13 shows a ¹H NMR spectrum of a benzotriazole compound synthesized in Synthesis Example 5 of the present application.
FIG. 14 shows a ¹H NMR spectrum of a benzotriazole compound synthesized in Synthesis Example 6 of the present application.
FIG. 15 shows a ¹H NMR spectrum of a benzotriazole compound synthesized in Synthesis Example 7 of the present application.
FIG. 16 shows a ¹H NMR spectrum of a benzotriazole compound synthesized in Synthesis Example 8 of the present application.
FIG. 17 shows I-V curves of solar cells containing two patterned anti-reflection layers according to a device example and a comparative example of the present application.
FIG. 18 shows external quantum efficiency vs. wavelength curves of solar cells containing two patterned anti-reflection layers according to a device example and a comparative example of the present application.

### DETAILED DESCRIPTION

The technical solutions of the embodiments of the present application will be described more clearly and comprehensively below in conjunction with the accompanying drawings for the embodiments of the present application. Apparently, the embodiments described herein are only part of, not all of the embodiments of the present application. Base on the embodiments of the present application, other embodiments obtained by those of ordinary skill in the art without creative efforts shall fall within the protection scope of the present application.

Unless defined otherwise, all technical and scientific terms used herein have the same meaning as commonly understood by those skilled in the art of the present application. The terms used in the specification of the present application are for the purpose of describing exemplary examples only and are not intended to limit the present application.

The terms "include", "have", and "contain" used in the present application indicates non-exclusive inclusion. Unless explicit limitation terms such as "only" and "consist of..." are used, another component can also be further added.

In the present application, the terms "first", "second", and "third" are used for descriptive purposes only, and cannot be construed as indicating or implying a relative importance, or implicitly specifying the number of the indicated technical features. Thus, the quantity of the feature defined with "first", "second" or "third" may explicitly or implicitly be at least one.

In the present application, the orientation or position relationships indicated by the terms "central", "longitudinal", "transverse", "length", "width", "thickness", "upper", "lower", "front", "back", "left", "right", "vertical", "horizontal", "top", "bottom", "inner", "outer", "clockwise", "counterclockwise" and the like are based on the orientational or positional relationships shown in the accompanying drawings and are intended to facilitate the description of the present application and simplify the description only, rather than indicating or implying that the apparatus or element referred to must have a particular orientation or be constructed and operated in a particular orientation, and therefore are not to be interpreted as limiting the present application.

As to description of the positional relationships, unless otherwise specified, when an element such as a layer, a film or a substrate is referred to as being "on" another layer or film, it may be directly on the other layer or film, or an intermediate layer or film may exist therebetween. Further, when a layer is referred to as being "under" another layer, it may be directly under the other layer, or one or more intermediate layers may exist therebetween. It can be also understood that when a layer is referred to as being "between" two layers, the layer may be the only layer between the two layers, or one or more intermediate layers may also exist therebetween.

In the present application, "further", "furthermore", "specially", etc., are for descriptive purposes, indicating the differences in content, but cannot be understood as a limitation on the protection scope of the present application.

In the present application, the term "at least one" means more than one, such as one, two, or more than two. The term "multiple" or "some" means at least two, such as two, three, etc. The term "multiple layers" means at least two layers, such as two layers, three layers, etc., unless otherwise explicitly and specifically defined. In the description of the present application, the term "several" means at least one, such as one, two, etc., unless otherwise explicitly and specifically defined.

Where a numerical range is disclosed in the present application, such a range is continuous, inclusive of both the minimum and maximum values of the range as well as every value between such minimum and maximum values. Further, where a range refers to integers, every integer between the minimum and maximum values of such a range is included. In addition, where multiple ranges are provided to describe a feature or characteristic, such ranges can be combined. That is to say that, unless otherwise indicated, all ranges disclosed herein are to be understood to encompass any and all sub-ranges subsumed therein. In addition, where only some specific numerical ranges are disclosed, any lower limit may be combined with any upper limit to form an unspecified range, any lower limit may be combined with any other lower limit to form an unspecified range, and any upper limit may be combined with any other upper limit to form an unspecified range. In addition, each separately disclosed point or single numerical value may be used as a lower limit or upper limit to combine with any other point or other single numerical value, or with any other lower or upper limit, to form an unspecified range.

Unless otherwise specified, all the steps of the present application can be performed sequentially or randomly. For example, the method including steps (a) and (b) indicates that the method may include steps (a) and (b) which are performed sequentially, and alternatively may include steps (b) and (a) which are performed sequentially. For example, when mentioning that the method may further include step (c), it indicates that step (c) may be added to the method in any order, e.g., the method may include steps (a), (b) and (c), or steps (a), (c) and (b), or steps (c), (a) and (b), etc.

Unless otherwise mentioned, a term in the singular form can include a plural form and cannot be understood as indicating a quantity of one.

In the present application, when a temperature parameter is mentioned, unless otherwise specifically stated, not only a thermostatic process but also a variation within a certain temperature interval is allowed. The thermostatic process allows for temperature fluctuations within the accuracy range of the instrument.

The weights of related components mentioned in the embodiments of the present application not only refer to specific contents of the components, but also represent proportional relationships between the weights of the components. Therefore, enlargement or reduction in scale according the contents of the related components in the embodiments of the present application should be within the scope disclosed by the embodiments of the present application. Specifically, the units of the weights described in the embodiments of the present application may be well known in the chemical industry, such as µg, mg, g, or kg.

In the present application, for unit of a numerical range, if the unit only appears after the right endpoint, it means that the units of both the left endpoint and the right endpoint are the same. For example, "800 to 850 nm" denotes that the unit nanometer (nm) applies to both the left endpoint "800" and the right endpoint "850".

In the present disclosure, the term "above" or "below" includes the number itself. For example, "below 1" means equal to or less than 1 (≤1), "above 1" means greater than or equal to 1(≥1).

In the present application, the phrase "A and B are each independently selected from x, y, or z" means that A and B are independent, where the selection of A does not affect the selection of B. Therefore, when A is selected from x, B can be selected from any one of x, y, or z; when A is selected from y, B can be selected from any one of x, y, or z; and when A is selected from z, B can be selected from any one of x, y, or z.

In the present application, "substituted or unsubstituted" means that the defined chemical group can be substituted or can be unsubstituted. When the defined chemical group is substituted, it should be understood as that the defined chemical group is substituted with any chemical group acceptable in the art, including, but not limited to: C₁ to C₃₀ alkyl, cycloalkyl having 3 to 20 ring atoms, heterocyclyl having 3 to 20 ring atoms, aryl having 5 to 20 ring atoms, heteroaryl having 5 to 20 ring atoms, a silane group, carbonyl, alkoxycarbonyl, aryloxycarbonyl, carbamoyl, haloformyl, formyl, -NRR', cyano, isocyano, an isocyanate group, a thiocyanate group, an isothiocyanate group, hydroxyl, trifluoromethyl, nitro, or halogen, and the above chemical groups can be further substituted with substituents acceptable in the art. It should be understood that R and R' in -NRR' are each independently substituted with a group acceptable in the art, including, but not limited to: H, C₁ to C₆ alkyl, cycloalkyl having 3 to 8 ring atoms, heterocyclyl having 3 to 8 ring atoms, aryl having 5 to 20 ring atoms, or heteroaryl having 5 to 10 ring atoms. The C₁ to C₆ alkyl, the cycloalkyl having 3 to 8 ring atoms, the heterocyclyl having 3 to 8 ring atoms, the aryl having 5 to 20 ring atoms, or the heteroaryl having 5 to 10 ring atoms are optionally further substituted with one or more of the following chemical groups: C₁ to C₆ alkyl, cycloalkyl having 3 to 8 ring atoms, heterocyclyl having 3 to 8 ring atoms, halogen, hydroxy, nitro, or amino.

In the present application, the term "the number of ring atoms" means the number of atoms constituting the ring itself in a structural compound (e.g., a monocyclic compound, a fused-ring compound, a cross-linked compound, a carbocyclic compound, or a heterocyclic compound) obtained by synthesizing the ring by atomic bonding. When the ring is substituted with a substituent, the atoms contained in the substituent are not included in the ring-forming atoms. The "number of ring atoms" mentioned below has the same meaning unless otherwise specified, for example, the number of ring atoms of benzene ring is 6, the number of ring atoms of naphthalene ring is 10, and the number of ring atoms of thienyl is 5.

In the present application, the term "alkyl" can represent a linear, branched, and/or cyclic alkyl group. The number of carbon atoms in alkyl can be in a range from 1 to 50, from 1 to 30, from 1 to 20, from 1 to 10, or from 1 to 6. The phrase containing this term, for example, "C₁ to C₉ alkyl" refers to an alkyl group containing 1 to 9 carbon atoms, and at each occurrence, can independently be C₁ alkyl, C₂ alkyl, C₃ alkyl, C₄ alkyl, C₅ alkyl, C₆ alkyl, C₇ alkyl, C₈ alkyl, or C₉ alkyl. Non-limiting examples of alkyl include methyl, ethyl, n-propyl, isopropyl, n-butyl, sec-butyl, tert-butyl, isobutyl, 2-ethylbutyl, 3,3-dimethylbutyl, n-pentyl, isopentyl, neopentyl, tert-pentyl, cyclopentyl, 1-methylpentyl, 3-methylpentyl, 2-ethylpentyl, 4-methyl-2-pentyl, n-hexyl, 1-methylhexyl, 2-ethylhexyl, 2-butylhexyl, cyclohexyl, adamantyl, and the like.

The term "alkoxy" refers to a chemical group having the structure of "-O-alkyl", where the alkyl as defined above is connected to the rest of the molecule via an oxygen atom. Examples of C₁ to C₂₀ alkoxy include C₁ to C₁₉ alkoxy, C₁ to C₁₄ alkoxy, C₁ to C₁₂ alkoxy, C₂ to C₆ alkoxy, C₂ to C₄ alkoxy, C₁₅ alkoxy, C₁₀ alkoxy, C₈ alkoxy, C₅ alkoxy, C₂₀ alkoxy, etc. Examples of the phrase containing the term "C₁ to C₂₀ alkoxy" include, but is not limited to: methoxy (-O-CH₃ or -OMe), ethoxy (-O-CH₂CH₃ or -OEt) and tert-butoxy (-O-C(CH)₃ or -OtBu).

The term "alkylthio" refers to an alkyl group connected to the rest of the molecule via a sulfur atom, i.e., "-S-alkyl", where "alkyl" is as defined above in the present application. Unless otherwise specified in the specification, alkylthio can be optionally substituted. Unless otherwise specified, the term "C₁ to C₂₀ alkylthio" refers to an alkyl group containing 1 to 20 carbon atoms connected to the rest of the parent molecule via a sulfur atom. Examples of C₁ to C₂₀ alkylthio include C₁ to C₁₉ alkylthio, C₁ to C₁₄ alkylthio, C₁ to C₁₂ alkylthio, C₂ to C₆ alkylthio, C₂ to C₄ alkylthio, C₁₅ alkylthio, C₁₀ alkylthio, C₈ alkylthio, C₅ alkylthio, C₂₀ alkylthio, etc. Specific examples of C₁ to C₂₀ alkylthio include, but are not limited to: -SCH₃, -SCH₂CH₃, -SCH₂CH₂CH₃, -SCH₂(CH3)₂, -SCH₂CH₂CH₂CH₃, -SCH₂CH₂(CH₃)₂, -SCH₂CH₂CH₂CH₂CH₃, -SCH₂CH₂CH₂CH₂CH₂CH₃, -SCH₂(CH₂CH₂CH₃)(CH₂CH₂CH₂CH₃).

The term "aryl", "aromatic group", or "aryl group" refers to an aromatic hydrocarbon group derived by removing one hydrogen atom from an aromatic cyclic compound, and can be monocyclic aryl, fused-ring aryl, or polycyclic aryl. For polycyclic aryl , at least one ring is an aromatic ring. For example, "substituted or unsubstituted aryl having 6 to 40 ring atoms" refers to substituted or unsubstituted aryl containing 6 to 40 ring atoms, preferably substituted or unsubstituted aryl having 6 to 30 ring atoms, more preferably substituted or unsubstituted aryl having 6 to 18 ring atoms, particularly preferably substituted or unsubstituted aryl having 6 to 14 ring atoms, and optionally the aryl is further substituted. Suitable examples include, but are not limited to: phenyl, biphenyl, terphenyl, naphthyl, anthryl, fluoranthenyl, phenanthrenyl, benzo[c]phenanthrenyl, perylenyl, tetracenyl, pyrenyl, benzo(a)pyrenyl, acenaphthylenyl, fluorenyl and their derivatives. It should be understood that multiple aromatic groups can be disrupted by short non-aromatic units (e.g. less than 10% non-H atoms such as C, N, or O atoms). Specific examples, such as acenaphthylenyl, fluorenyl, 9,9-diarylfluorenyl, triarylamino, and diarylether systems should also be included in the definition of aryl.

In the present application, the term "heteroaryl", "heteroaryl group", or "heteroaromatic group" refers to an aryl group in which at least one carbon atom is replaced by a non-carbon atom. The non-carbon atom can be an N atom, an O atom, or an S atom. For example, "substituted or unsubstituted heteroaryl having 5 to 40 ring atoms" refers to substituted or unsubstituted heteroaryl containing 5 to 40 ring atoms, preferably substituted or unsubstituted heteroaryl having 6 to 30 ring atoms, more preferably substituted or unsubstituted heteroaryl having 6 to 18 ring atoms, particularly preferably substituted or unsubstituted heteroaryl having 6 to 14 ring atoms, and optionally the heteroaryl is further substituted. Suitable examples include, but are not limited to: triazinyl, pyridyl, pyrimidyl, imidazolyl, furanyl, thienyl, benzofuranyl, benzothiophenyl, indolyl, carbazolyl, pyrroloimidazolyl, pyrrolopyrrolyl, thienopyrrolyl, thienothiophenyl, furopyrrolyl, furofuranyl, thienofuranyl, benzoisoxazolyl, benzoisothiazolyl, benzimidazolyl, quinolinyl, isoquinolinyl, cinnolino, quinoxalinyl, phenanthridinyl, perimidinyl, quinazolinyl, quinazolinonyl, dibenzothiophenyl, dibenzofuranyl, carbazolyl, and derivatives thereof.

In the present application, the "*" connected to a single bond represents a linking site.

The present application provides a benzotriazole organic compound, a preparation method thereof, and use thereof, aiming to provide a new functional organic material with good stability, which can improve the photovoltaic efficiency and service life of photovoltaic devices.

The technical solutions are as follows:

A benzotriazole organic compound has a general structure formula represented by formula (1): 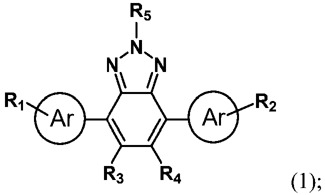
wherein each Ar independently includes a substituted or unsubstituted aryl, or a substituted or unsubstituted heteroaryl;
R₁ and R₂ are each independently selected from substituted or unsubstituted alkyl, or substituted or unsubstituted alkoxy;
R₃ and R₄ are each independently selected from halogen, substituted or unsubstituted alkoxy, or substituted or unsubstituted alkylthio; and
R₅ is selected from substituted or unsubstituted alkyl.

The benzotriazole organic compound provided in the present application is benzotriazole modified with specific Ar, R₁, R₂, R₃, R₄, and R₅ groups, which can enhance the fluorescence properties of the product, shift the absorption spectrum to the ultraviolet light region, and shift the fluorescence emission spectrum to the visible light region. Moreover, the introduction of R₃ and R₄ can modulate the donor-acceptor interactions within the molecule, increase molecular rigidity, raise the oxidation barrier, and improve molecular stability. Additionally, the increased molecular rigidity can also enhance the absorption and extinction coefficients and fluorescence quantum yield of the compound, thereby improving light conversion efficiency and the conversion efficiency of photovoltaic devices.

Through the synergy of benzotriazole and the various substituents, the benzotriazole organic compound can absorb ultraviolet light and convert it into visible light, such as blue light or sky-blue light, while exhibiting good stability, high absorption and extinction coefficients, and a high fluorescence quantum yield. The benzotriazole organic compound can serve as a light conversion agent. A photovoltaic encapsulant film made of the benzotriazole organic compound can offer advantages such as excellent light conversion effect, high stability, and low water-vapor transmission rate. When further applied in photovoltaic devices, it can improve the photovoltaic efficiency and stability of the photovoltaic devices, and extend the service life of the devices.

Testing has shown that, in some embodiments of the present application, the benzotriazole organic compound has a light absorption in a wavelength range from 280 nm to 400 nm, a maximum absorption peak at 320 nm to 380 nm, a photoluminescence in a range from 400 nm to 600 nm, and a maximum emission peak at 450 nm to 550 nm. When applied in photovoltaic encapsulant films and subsequently used in photovoltaic devices, the benzotriazole organic compound can increase the photovoltaic efficiency of the photovoltaic devices by more than 1% and extend the service life by more than 50%.

In the present application, each Ar independently includes substituted or unsubstituted aryl, or substituted or unsubstituted heteroaryl. Further, each Ar independently includes substituted or unsubstituted aryl having 6 to 30 ring atoms, or substituted or unsubstituted heteroaryl having 5 to 30 ring atoms. Furthermore, each Ar independently includes substituted or unsubstituted aryl having 6 to 20 ring atoms, or substituted or unsubstituted heteroaryl having 5 to 20 ring atoms. Specifically, each Ar is independently selected from substituted or unsubstituted six-membered aryl, substituted or unsubstituted six-membered heteroaryl, or substituted or unsubstituted five-membered heteroaryl.

In some embodiments, each Ar is independently selected from one of the following groups: 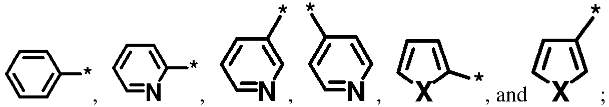 wherein * represents a linking site, and X represents O, S, or Se.

In some embodiments, each Ar is independently selected from one of the following groups: 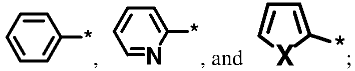 wherein * represents a linking site, and X represents O, S, or Se.

In the present application, R₁ and R₂ are each independently selected from substituted or unsubstituted alkyl, or substituted or unsubstituted alkoxy. Further, R₁ and R₂ are each independently selected from substituted or unsubstituted C₁ to C₂₀ alkyl, or substituted or unsubstituted C₁ to C₂₀ alkoxy. Optionally, R₁ and R₂ are each independently selected from substituted or unsubstituted C₁ to C₂₀ linear alkyl, substituted or unsubstituted C₃ to C₂₀ branched alkyl, substituted or unsubstituted C₃ to C₂₀ cycloalkyl, substituted or unsubstituted C₁ to C₂₀ linear alkoxy, substituted or unsubstituted C₃ to C₂₀ branched alkoxy, or substituted or unsubstituted C₃ to C₂₀ cycloalkoxy.

In some embodiments, R₁ and R₂ are each independently selected from C₁ to C₂₀ linear alkyl substituted or unsubstituted with R, C₃ to C₂₀ branched alkyl substituted or unsubstituted with R, C₁ to C₂₀ linear alkoxy substituted or unsubstituted with R, or C₃ to C₂₀ branched alkoxy substituted or unsubstituted with R. Further, R₁ and R₂ are each independently selected from C₁ to C₁₀ linear alkyl substituted or unsubstituted with R, C₃ to C₁₀ branched alkyl substituted or unsubstituted with R, or C₁ to C₁₀ linear alkoxy substituted or unsubstituted with R, or C₃ to C₁₀ branched alkoxy substituted or unsubstituted with R. R in R₁ and R₂ is each independently C₁ to C₆ linear alkyl, and further, R in R₁ and R₂ is methyl, ethyl, n-propyl, or n-butyl.

In some embodiments, R₁ and R₂ are each independently selected from one of the following groups: 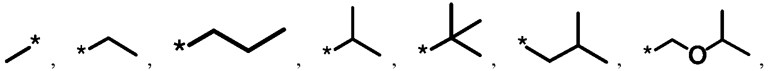 and 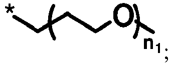 wherein * represents a linking site, and n1≥2.

In the present application, R₃ and R₄ are each independently selected from halogen (i.e., an element of the seventh main group), substituted or unsubstituted alkoxy, or substituted or unsubstituted alkylthio. Optionally, R₃ and R₄ are each independently selected from halogen, substituted or unsubstituted C₁ to C₂₀ alkoxy, or substituted or unsubstituted C₁ to C₂₀ alkylthio. Specifically, R₃ and R₄ are each independently selected from fluorine (F), chlorine (Cl), bromine (Br), iodine (I), astatine (At), substituted or unsubstituted C₁ to C₂₀ linear alkoxy, substituted or unsubstituted C₃ to C₂₀ branched alkoxy, substituted or unsubstituted C₃ to C₂₀ cycloalkoxy, substituted or unsubstituted C₁ to C₂₀ linear alkylthio, substituted or unsubstituted C₃ to C₂₀ branched alkylthio, or substituted or unsubstituted C₃ to C₂₀ cyclic alkylthio.

Preferably, R₃ and R₄ are each independently selected from one of the following groups: 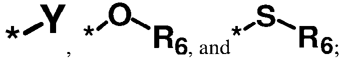 wherein * represents a linking site, Y represents F, Cl, Br, or I, and each R₆ is independently selected from substituted or unsubstituted C₁ to C₂₀ linear alkyl, or substituted or unsubstituted C₃ to C₂₀ branched alkyl.

Further, R₃ and R₄ are each independently selected from one of the following groups:

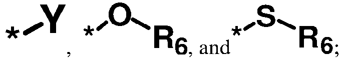

Each R₆ is independently selected from C₁ to C₂₀ linear alkoxy substituted or unsubstituted with R, C₃ to C₂₀ branched alkoxy substituted or unsubstituted with R, C₁ to C₂₀ linear alkylthio substituted or unsubstituted with R, or C₃ to C₂₀ branched alkylthio substituted or unsubstituted with R. Further, each R₆ is independently selected from C₁ to C₁₀ linear alkoxy substituted or unsubstituted with R, C₃ to C₁₀ branched alkoxy substituted or unsubstituted with R, C₁ to C₁₀ linear alkylthio substituted or unsubstituted with R, or C₃ to C₁₀ branched alkylthio substituted or unsubstituted with R.

Optionally, R in R₆ is independently C₁ to C₂₀ linear alkyl, or C₃ to C₂₀ linear alkyl, or is further independently C₁ to C₂₀ linear alkyl, C₃ to C₂₀ branched alkyl, or C₃ to C₂₀ cyclic alkyl. Further, R in R₆ is independently C₁ to C₁₀ linear alkyl, C₃ to C₁₀ branched alkyl, or C₃ to C₁₀ cycloalkyl. Furthermore, R in R₆ is independently C₁ to C₆ linear alkyl or C₃ to C₆ branched alkyl. Specifically, R in R₆ is independently methyl, ethyl, n-propyl, or n-butyl.

In some embodiments, R₃ and R₄ are each independently selected from one of the following groups: 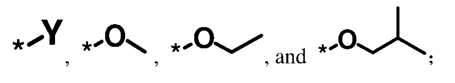 wherein * represents a linking site, Y represents F, Cl, Br, or I.

In the present application, R₅ is selected from substituted or unsubstituted alkyl. Preferably, R₅ is selected from substituted or unsubstituted C₁ to C₂₀ alkyl. Optionally, R₅ is selected from substituted or unsubstituted C₁ to C₂₀ linear alkyl, substituted or unsubstituted C₃ to C₂₀ branched alkyl, or substituted or unsubstituted C₃ to C₂₀ cycloalkyl. Preferably, R₅ is selected from C₁ to C₂₀ linear alkyl substituted or unsubstituted with R, or C₃ to C₂₀ branched alkyl substituted or unsubstituted with R, or C₃ to C₂₀ cycloalkyl substituted or unsubstituted with R. Further, R₅ is independently selected from C₁ to C₁₀ linear alkyl substituted or unsubstituted with R, C₃ to C₁₀ branched alkyl substituted or unsubstituted with R, or C₃ to C₁₀ cycloalkyl substituted or unsubstituted with R.

Optionally, R in R₅ is independently C₁ to C₂₀ linear alkyl, or C₃ to C₂₀ linear alkyl, or is further independently C₁ to C₂₀ linear alkyl, C₃ to C₂₀ branched alkyl, or C₃ to C₂₀ cycloalkyl. Further, R in R₅ is independently C₁ to C₁₀ linear alkyl, C₃ to C₁₀ branched alkyl, or C₃ to C₁₀ cycloalkyl. Furthermore, R in R₅ is independently C₁ to C₆ linear alkyl or C₃ to C₆ branched alkyl. Specifically, R in R₅ is independently methyl, ethyl, n-propyl, or n-butyl.

In some embodiments, R₅ is selected from one of the following groups: 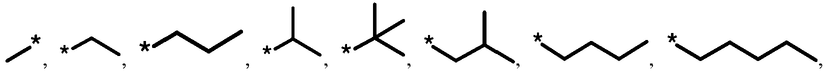 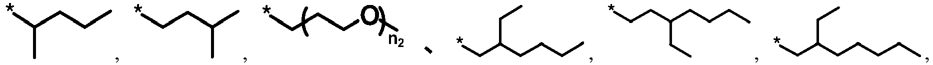 and 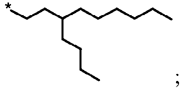 wherein * represents a linking site, and n₂≥2.

In some embodiments, the benzotriazole organic compound of the present application has any one of the following structures:

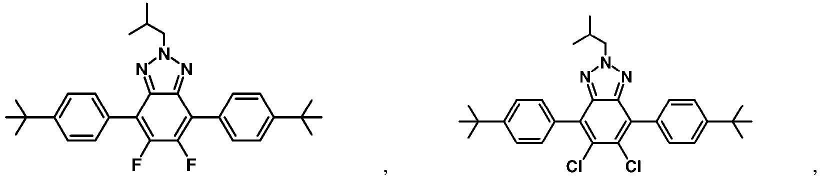

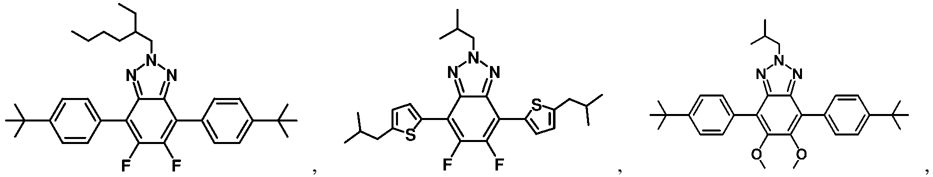

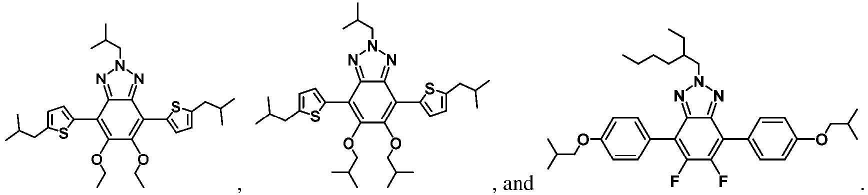

The present application further provides a method for preparing the benzotriazole organic compound as described above, which includes the following steps:
mixing and reacting compound (a), compound (b), compound (c), and an alkali in a solvent to prepare the benzotriazole organic compound represented by formula (1);

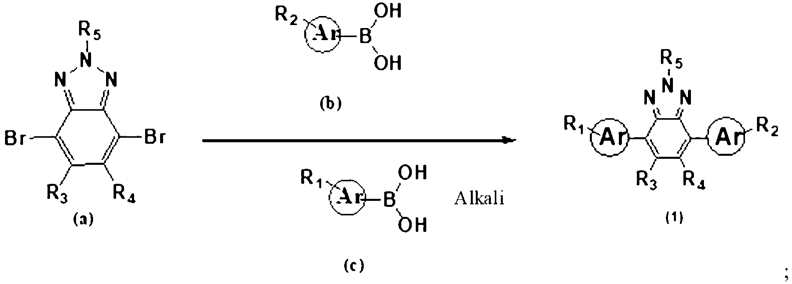

The definitions of R₁, R₂, R₃, R₄, and R₅ are the same as those previously described for the benzotriazole organic compound.

In some embodiments, a molar ratio of compound (a), compound (b), and compound (c) is substantially 1:(1 to 2):(1 to 2).

In some embodiments, a molar ratio of the alkali to compound (a) is substantially (1 to 2):1.

In some embodiments, the alkali is one or more selected from potassium carbonate, sodium carbonate, sodium methoxide, sodium ethoxide, and sodium tert-butoxide.

In some embodiments, the solvent includes a mixed solvent of 1,4-dioxane and water.

In some embodiments, the reaction temperature is in a range from about 80 °C to about 100 °C, and the reaction time period is in a range from about 20 hours to about 40 hours.

In some embodiments, the method for preparing the benzotriazole organic compound as described above includes the following steps:
dissolving a 4,7-dibromobenzotriazole derivative represented by formula (a) and alkylphenylboronic acids represented by formulas (b) and (c) in 1,4-dioxane, and adding an alkali and deionized water; then replacing the reaction atmosphere with an inert gas (nitrogen or argon), heating the mixture to a temperature ranged from about 80 °C to about 100 °C, and reacting for about 20 hours to about 40 hours; after cooling to room temperature, quenching the system with water and extracting with dichloromethane, spin-drying, and purifying by column chromatography to obtain a target product, which is a white powder.

In some embodiments, the method for preparing the benzotriazole organic compound as described above includes the following steps:
dissolving about 1 mmol of a 4,7-dibromobenzotriazole derivative and about 3 mmol of 4-alkylphenylboronic acid in about 10 ml of 1,4-dioxane, and adding about 2 mmol of potassium carbonate and about of 1 ml of deionized water; then replacing the reaction atmosphere with an inert gas (nitrogen or argon), heating the mixture to about 95 °C, and reacting for about 24 hours; after cooling to room temperature, quenching the system with water and extracting with dichloromethane, spin-drying, and purifying by column chromatography to obtain a target product, which is a white powder.

The present application further provides use of the benzotriazole organic compound as described above. The technical solutions are as follows:

A light conversion agent includes the benzotriazole organic compound as described above.

In some embodiments, the light conversion agent has a light absorption in a wavelength range from about 280 nm to about 400 nm.

In some embodiments, the light conversion agent has a maximum absorption peak at about 320 nm to about 380 nm.

In some embodiments, the light conversion agent has a photoluminescence in a range from about 400 nm to about 600 nm.

In some embodiments, the light conversion agent has a maximum emission peak at about 450 nm to about 550 nm.

A photovoltaic encapsulant film includes the benzotriazole organic compound as described above, or includes the light conversion agent as described above. The photovoltaic encapsulant film can absorb ultraviolet light and convert the ultraviolet light into visible light, such as blue light or sky-blue light, while exhibiting good stability, high absorption and extinction coefficients, and a high fluorescence quantum yield. The photovoltaic encapsulant film can offer advantages such as excellent light conversion effect, high stability, and low water-vapor transmission rate, and when further applied in photovoltaic devices, can improve the photovoltaic efficiency and stability of the photovoltaic devices, and extend the service life of the devices.

In some embodiments, the photovoltaic encapsulant film further includes an optically transparent polymer material. It should be understood that, in the present application, the optically transparent polymer material refers to a polymer material with a light transmittance equal to or greater than 85% for visible light, a refractive index of about 1.2 to about 1.7, and a neutral density of about 0.75 g/cm³ to about 1.2 g/cm³. In some embodiments, the optically transparent polymer material described in the present application has a light transmittance equal to or greater than about 90% for visible light, a refractive index of about 1.4 to about 1.6, and a neutral density of about 0.75 g/cm³ to about 1.2 g/cm³.

In some embodiments, the photovoltaic encapsulant film includes, by mass percentage, about 0.01% to about 5% of the benzotriazole organic compound as described above or the light conversion agent as described above, and about 95% to about 99.99% of the polymer material.

In some embodiments, the polymer material is one or more selected from the group consisting of a polyolefin elastomer (POE), an ethylene-vinyl acetate copolymer (EVA), polyvinyl butyral (PVB), and an organic silicone resin material. Further, the organic silicone resin material is one or more selected from a deacidified one-component silicone, a dealcoholized one-component silicone, a deoximated one-component silicone, a hydrogen-containing silicone, and a vinyl-terminated polydimethylsiloxane.

In some embodiments, the organic silicone resin material includes a mixture of a hydrogen-containing silicone and a vinyl-terminated polydimethylsiloxane in a mass ratio of substantially 1:(2 to 15). Such a photovoltaic encapsulant film exhibits both anti-reflection and light-conversion functions. Optionally, the hydrogen-containing silicone includes one or more of DC182, DC184, and DC186 from Dow and Fuletonsan 2538.

It should be understood that, the photovoltaic encapsulant film further includes an additive. Further, the additive includes one or more selected from an initiator, an antioxidant, a silane coupling agent, and a light stabilizer.

In some embodiments, the photovoltaic encapsulant film includes, by mass percentage, about 0.01% to 4.95% of the benzotriazole organic compound as described above or the light conversion agent as described above, about 95% to about 99.94% of the polymer material, and about 0.05% to about 4.99% of the additive.

In some embodiments, the photovoltaic encapsulant film includes, by mass percentage, about 0.01% to about 4.85% of the benzotriazole organic compound as described above or the light conversion agent as described above, about 95% to about 99.84% of the polymer material, about 0.05% to about 1.5% of the initiator, about 0.05% to about 1.5% of the silane coupling agent, and about 0.05% to about 1.5% of the antioxidant.

In some embodiments, the photovoltaic encapsulant film includes, by mass percentage, about 0.01% to about 4.95% of the benzotriazole organic compound as described above or the light conversion agent as described above, about 95% to about 99.94% of the polymer material, and about 0.05% to about 3% of the antioxidant.

The present application further provides a photovoltaic device including a solar cell and the photovoltaic encapsulant film as described above, wherein the photovoltaic encapsulant film is disposed on at least one surface of the solar cell. Optionally, surfaces of the solar cell are coated with the photovoltaic encapsulant film as described above. Alternatively, the photovoltaic encapsulant film is disposed on at least one surface of at least one functional layer of the solar cell.

In some embodiments, the solar cell includes a crystalline silicon cell, a perovskite cell, a tandem cell including the perovskite cell and the crystalline silicon cell, or any combination thereof.

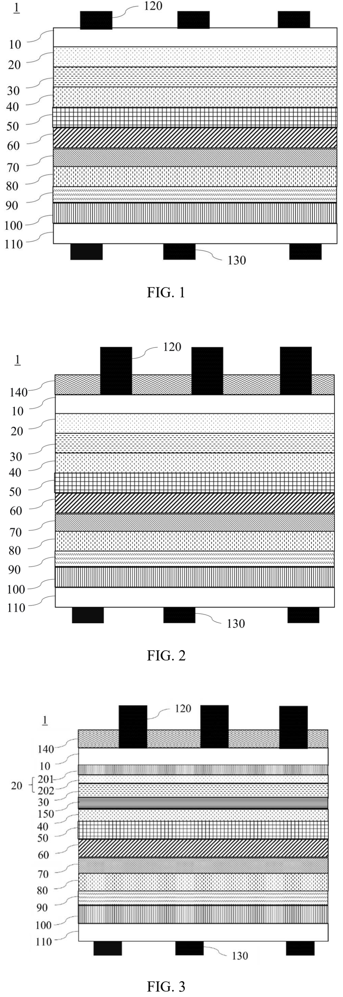

In some embodiments, the solar cell is the tandem cell including the perovskite cell and the crystalline silicon cell, the perovskite cell constitutes a light-receiving surface, and crystalline silicon cell constitutes a back surface. Further, referring to FIG. 1, the solar cell 1 includes a first transparent conductive layer 10, an electron transport layer 20, a perovskite active layer 30, a hole transport layer 40, a second transparent conductive layer 50, an N-type doped layer 60, a first amorphous silicon layer 70, a silicon wafer 80 (also known as a silicon substrate), a second amorphous silicon layer 90, a P-type doped layer 100, and a third transparent conductive layer 110 stacked in sequence, and further includes a first finger electrode layer and a second finger electrode layer.

The first finger electrode layer includes a plurality of first finger electrodes 120 spaced apart from each other, and each first finger electrode 120 is electrically connected to the first transparent conductive layer 10.

The second finger electrode layer includes a plurality of second finger electrodes 130 spaced apart from each other, and each second finger electrode 130 is electrically connected to the third transparent conductive layer 110.

In some embodiments, in the present application, the electrical connection refers to a physical contact, meaning that the electrical connection is a circuit connection established through physical contact rather than a signal connection through wireless communication.

In the present application, optionally, the material of the first transparent conductive layer 10 includes, but is not limited to, one or more selected from the group consisting of indium tin oxide, aluminum zinc oxide, aluminum indium oxide, indium cerium oxide, and indium tungsten oxide. Optionally, the thickness of the first transparent conductive layer 10 is in a range from about 5 nm to about 200 nm.

In the present application, optionally, the material of the perovskite active layer 30 includes, but is not limited to, a compound with an ABX₃ structure, where A is one or more selected from a methylammonium cation, a formamidinium cation, and Cs⁺; B is one or more selected from Sn²⁺ and Pb²⁺; and X is one or more selected from F⁻, Cl⁻, Br⁻, and I⁻. Optionally, the thickness of the perovskite active layer 30 is in a range from about 0.6 µm to about 2 µm.

In the present application, optionally, the material of the hole transport layer 40 includes, but is not limited to, one or more selected from the group consisting of nickel oxide, copper oxide, and molybdenum oxide. Optionally, the thickness of the hole transport layer 40 is in a range from about 5 nm to about 20 nm.

In the present application, optionally, the material of the second transparent conductive layer 50 includes, but is not limited to, one or more selected from the group consisting of indium tin oxide, aluminum zinc oxide, aluminum indium oxide, indium cerium oxide, and indium tungsten oxide. Optionally, the thickness of the second transparent conductive layer 50 is in a range from about 5 nm to about 40 nm.

In the present application, optionally, the material of the N-type doped layer 60 includes, but is not limited to, one or more selected from the group consisting of phosphorus-doped amorphous silicon, phosphorus-doped nano silicon (nanocrystalline silicon or microcrystalline silicon), and phosphorus-doped amorphous silicon oxycarbide. Optionally, the thickness of the N-type doped layer 60 is in a range from about 3 nm to about 10 nm.

In the present application, optionally, the material of the first amorphous silicon layer 70 includes, but is not limited to, one or more selected from the group consisting of hydrogenated amorphous silicon, hydrogenated silicon oxide, and hydrogenated silicon oxynitride. Optionally, the thickness of the first amorphous silicon layer 70 is in a range from about 1 nm to about 2 nm.

In the present application, optionally, the thickness of the silicon wafer 80 is in a range from about 200 µm to about 500 µm.

In the present application, optionally, the material of the second amorphous silicon layer 90 includes, but is not limited to, one or more selected from the group consisting of hydrogenated amorphous silicon, hydrogenated silicon oxide, and hydrogenated silicon oxynitride. Optionally, the thickness of the second amorphous silicon layer 90 is in a range from about 1 nm to about 2 nm.

In the present application, optionally, the material of the P-type doped layer 100 includes, but is not limited to, one or more selected from the group consisting of boron-doped amorphous silicon, boron-doped nano silicon (nanocrystalline silicon or microcrystalline silicon), and boron-doped amorphous silicon oxycarbide. Optionally, the thickness of the P-type doped layer 100 is in a range from about 3 nm to about 20 nm.

In the present application, optionally, the material of the third transparent conductive layer 110 includes, but is not limited to, one or more selected from the group consisting of indium tin oxide, aluminum zinc oxide, aluminum indium oxide, indium cerium oxide, and indium tungsten oxide. Optionally, the thickness of the third transparent conductive layer 110 is in a range from about 5 nm to about 200 nm.

Referring to FIG. 2, in some embodiments, the solar cell 1 further includes a first anti-reflection layer 140. The anti-reflection layer 140 is stacked on the surface of the first transparent conductive layer 10 away from the perovskite active layer 30. The first finger electrodes extend through the first anti-reflection layer 140 and are electrically connected to the first transparent conductive layer 10.

Optionally, the material of the first anti-reflection layer 140 includes magnesium fluoride, lithium fluoride, silicon nitride, silicon oxide, silicon oxynitride, or any combination thereof.

In the present application, optionally, the thickness of the first anti-reflection layer 140 is in a range from about 20 nm to about 200 nm.

It should be understood that, in the present application, the electron transport layer 20 can include either a single layer or two layers. For example, in the direction approaching the perovskite active layer 30, the electron transport layer 20 can include a first electron transport layer 201 and a second electron transport layer 202. The material of the first electron transport layer 201 includes, but is not limited to, one or more semiconductor metal oxides selected from tin oxide, zinc oxide, and titanium oxide. The material of the second electron transport layer 202 includes, but is not limited to, one or more fullerene derivatives selected from C₆₀, C₇₀, [6,6]-phenyl-C₆₁-butyric acid methyl ester (PC₆₁BM), [6,6]-phenyl-C₇₁-butyric acid methyl ester (PC₇₁BM), or one or more electron transport materials selected from a naphthalene diimide or a perylene diimide.

It should be understood that, a hole modification layer 150 can be further disposed between the perovskite active layer 30 and the hole transport layer 40 to further enhance hole transport capability. Further, the material of the hole modification layer 150 includes, but is not limited to, one or more self-assembling materials containing carbazole and phosphonic acid functional groups selected from (4-(3,6-dimethyl-9H-carbazol-9-yl)butyl)phosphonic acid (Me-4PACz), (2-(3,6-dimethoxy-9H-carbazol-9-yl)ethyl)phosphonic acid (MeO-2PACz), and (2-(9H-carbazol-9-yl)ethyl)phosphonic acid (2PACz).

It should be understood that, the N-type doped layer 60 can be an N-type microcrystalline silicon layer or an N-type amorphous silicon layer, and the P-type doped layer 100 can be a P-type microcrystalline silicon layer or a P-type amorphous silicon layer. The first amorphous silicon layer 70 and the second amorphous silicon layer 90 are each independently an intrinsic hydrogenated amorphous silicon layer.

Referring to FIG. 3, in an embodiment of the present application, the tandem cell includes a perovskite cell and a crystalline silicon cell, specifically including a first anti-reflection layer 140, a first transparent conductive layer 10, a first electron transport layer 201, a second electron transport layer 202, a perovskite active layer 30, a hole modification layer 150, a hole transport layer 40, a second transparent conductive layer 50, an N-type doped layer 60, a first amorphous silicon layer 70, a silicon wafer 80, a second amorphous silicon layer 90, a P-type doped layer 100, and a third transparent conductive layer 110 stacked in sequence, and further includes a first finger electrode layer and a second finger electrode layer.

The first finger electrode layer includes a plurality of first finger electrodes 120 spaced apart from each other, and each first finger electrode 120 is electrically connected to the first transparent conductive layer 10.

The second finger electrode layer includes a plurality of second finger electrodes 130 spaced apart from each other, and each second finger electrode 130 is electrically connected to the third transparent conductive layer 110.

In some embodiments, in the present application, the electrical connection refers to a physical contact, meaning that the electrical connection is a circuit connection established through a physical contact rather than a signal connection through wireless communication.

Further, in the tandem cell shown in FIG. 3, the material of the first anti-reflection layer 140 includes magnesium fluoride, lithium fluoride, silicon nitride, silicon oxide, silicon oxynitride, or any combination thereof.

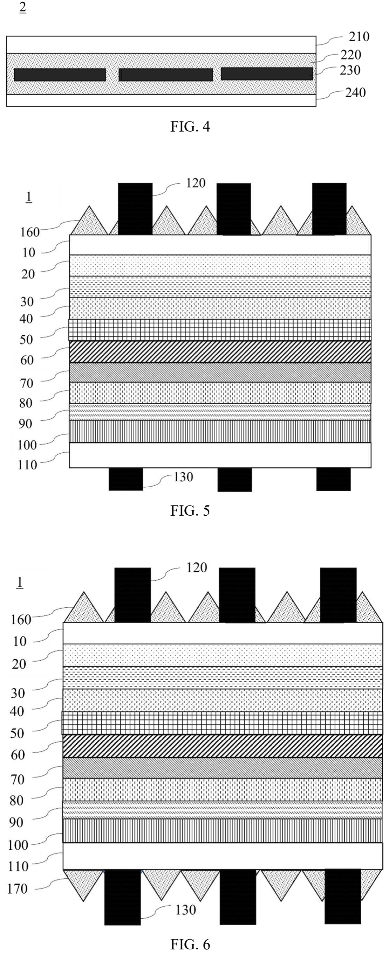

Referring to FIG. 4, the present application further provides a photovoltaic device, including a light-receiving surface plate 210, a solar cell 230, and a back surface plate 240 which are sequentially stacked, wherein surfaces of the solar cell are coated with the photovoltaic encapsulant film 220 as described above. Furthermore, the solar cell can have a structure shown in FIG. 1, FIG. 2, or FIG. 3.

It should be understood that in the present application, the light-receiving surface is also referred to as a light-incident surface.

In some embodiments, the light-receiving surface plate 210 and the back surface plate 240 are glass plates.

The present application further provides a method for preparing the photovoltaic device as shown in FIG. 4, including:
selecting a front glass plate with a desired size, cutting an encapsulant film containing a light conversion agent to match the size, and sequentially stacking the cut encapsulant film, a string of electrically connected cells, a second encapsulant film, and a back glass plate on the front glass plate, and laminating the stack in a laminator to obtain a laminated module; subsequently, installing a junction box and a metal frame to obtain the photovoltaic device as shown in FIG. 4.

In the industry, anti-reflection technologies for perovskite typically employ vapor-deposited MgFₓ (non-stoichiometric MgF₂, where optionally, 1.5≤x≤2) films. However, MgFₓ has a relatively low yield and relatively high cost, which is not conducive to mass production. In addition, for achieving light conversion, a light conversion agent is commonly added into the ethylene-vinyl acetate copolymer (EVA) or polyolefin elastomer (POE) encapsulant films in the industry. The anti-reflection technology and light conversion technology are two independently developed systems with no precedent for combination. In some embodiments of the present application, the photovoltaic anti-reflection technology is combined with the light conversion technology. This approach reduces photon reflection at the cell surface, thereby increasing transmittance to improve device power, while also reducing damage to the cell from ultraviolet light and converting ultraviolet light into visible light to further enhance device power. Additionally, the anti-reflection materials used are abundant in source and can be easily introduced into mass production for industrial applications.

The technical solutions are as follows:
A photovoltaic device includes a solar cell and the photovoltaic encapsulant film as described above, wherein the photovoltaic encapsulant film is patterned and disposed on at least one surface of the solar cell. Further, the photovoltaic encapsulant film is disposed in the anti-reflection layer of the solar cell, serving both anti-reflection and light-conversion functions. Further, the anti-reflection layer includes about 0.01% to about 5% of the benzotriazole organic compound or the light conversion agent as described in the present application, and about 95% to about 99.99% of the organic silicone resin material, wherein the organic silicone resin material is a mixture of a hydrogen-containing silicone and a vinyl-terminated polydimethylsiloxane in a mass ratio of substantially 1:(2 to 15). Such an anti-reflection layer with a photovoltaic encapsulant film exhibits both anti-reflection and light-conversion functions and can be referred to as an anti-reflection light-conversion photovoltaic encapsulant film.

In some embodiments, the solar cell includes a crystalline silicon cell, a perovskite cell, a tandem cell including the perovskite cell and the crystalline silicon cell, or any combination thereof.

In some embodiments, the solar cell is the tandem cell including the perovskite cell and the crystalline silicon cell, and the perovskite cell constitutes a light-receiving surface and the crystalline silicon cell constitutes a back surface. Further, referring to FIG. 5, a solar cell includes a second anti-reflection layer 160, a first transparent conductive layer 10, an electron transport layer 20, a perovskite active layer 30, a hole transport layer 40, a second transparent conductive layer 50, an N-type doped layer 60, a first amorphous silicon layer 70, a silicon wafer 80, a second amorphous silicon layer 90, a P-type doped layer 100, and a third transparent conductive layer 110 stacked in sequence, and further includes a first finger electrode layer and a second finger electrode layer.

The first finger electrodes 120 extend through the second anti-reflection layer 160 and are electrically connected to the first transparent conductive layer 10.

The second finger electrodes 130 are electrically connected to the third transparent conductive layer 110.

The second anti-reflection layer 160 includes a patterned photovoltaic encapsulant film as described above. The photovoltaic encapsulant film includes about 0.01% to about 5% of the benzotriazole organic compound or the light conversion agent, and about 95% to about 99.99% of the organic silicone resin material. The organic silicone resin material is a mixture of a hydrogen-containing silicone and a vinyl-terminated polydimethylsiloxane in a mass ratio of substantially 1:(2 to 15).

Further, referring to FIG. 6, a solar cell includes a second anti-reflection layer 160, a first transparent conductive layer 10, an electron transport layer 20, a perovskite active layer 30, a hole transport layer 40, a second transparent conductive layer 50, an N-type doped layer 60, a first amorphous silicon layer 70, a silicon wafer 80, a second amorphous silicon layer 90, a P-type doped layer 100, a third transparent conductive layer 110, and a third anti-reflection layer 170 stacked in sequence, and further includes a first finger electrode layer and a second finger electrode layer.

The first finger electrodes 120 extend through the second anti-reflection layer 160 and are electrically connected to the first transparent conductive layer 10.

The second finger electrodes 130 extend through the third anti-reflection layer 170 and are electrically connected to the third transparent conductive layer 110.

The second anti-reflection layer 160 includes a patterned photovoltaic encapsulant film as described above. The photovoltaic encapsulant film includes about 0.01% to about 5% of the benzotriazole organic compound or the light conversion agent, and about 95% to about 99.99% of the organic silicone resin material. The organic silicone resin material is a mixture of a hydrogen-containing silicone and a vinyl-terminated polydimethylsiloxane in a mass ratio of substantially 1:(2 to 15).

The third anti-reflection layer 160 includes a patterned photovoltaic encapsulant film as described above. The photovoltaic encapsulant film includes about 0.01% to about 5% of the benzotriazole organic compound or the light conversion agent, and about 95% to about 99.99% of the organic silicone resin material. The organic silicone resin material is a mixture of a hydrogen-containing silicone and a vinyl-terminated polydimethylsiloxane in a mass ratio of substantially 1:(2 to 15).

It should be understood that the second anti-reflection layer 160 and the third anti-reflection layer 170 are independent from each other, and their respective compositions can be the same or different. That is, the types of benzotriazole organic compounds in the second anti-reflection layer 160 and the third anti-reflection layer 170 can be the same or different, and the contents of benzotriazole organic compounds can be the same or different. Similarly, the types of organic silicone resin materials can be the same or different, and the contents of organic silicone resin materials can be the same or different. The types of hydrogen-containing silicones can be the same or different, the types of vinyl-terminated polydimethylsiloxanes can be the same or different, and the mass ratios of the hydrogen-containing silicone to the vinyl-terminated polydimethylsiloxane can be the same or different.

In the present application, for the photovoltaic device where the anti-reflection layer includes a patterned anti-reflection light-conversion photovoltaic encapsulant film, optionally, the material of the first transparent conductive layer includes, but is not limited to, one or more selected from the group consisting of indium tin oxide, aluminum zinc oxide, aluminum indium oxide, indium cerium oxide, and indium tungsten oxide. Optionally, the thickness of the first transparent conductive layer is in a range from about 5 nm to about 200 nm. The material of the perovskite active layer includes, but is not limited to, a compound with a ABX₃ structure, where A is one or more selected from a methylammonium cation, a formamidinium cation, and Cs⁺; B is one or more selected from Sn²⁺ and Pb²⁺; and X is one or more selected from F⁻, Cl⁻, Br⁻, and I⁻. Optionally, the thickness of the perovskite active layer is in a range from about 0.6 µm to about 2 µm. The material of the hole transport layer includes, but is not limited to, one or more selected from the group consisting of nickel oxide, copper oxide, and molybdenum oxide. Optionally, the thickness of the hole transport layer is in a range from about 5 nm to about 20 nm. The material of the second transparent conductive layer includes, but is not limited to, one or more selected from the group consisting of indium tin oxide, aluminum zinc oxide, aluminum indium oxide, indium cerium oxide, and indium tungsten oxide. Optionally, the thickness of the second transparent conductive layer is in a range from about 5 nm to about 40 nm. The material of the N-type doped layer includes, but is not limited to, one or more selected from the group consisting of phosphorus-doped amorphous silicon, phosphorus-doped nano silicon (nanocrystalline silicon or microcrystalline silicon), and phosphorus-doped amorphous silicon oxycarbide. Optionally, the thickness of the N-type doped layer is in a range from about 3 nm to about 10 nm. The material of the first amorphous silicon layer includes, but is not limited to, one or more selected from the group consisting of hydrogenated amorphous silicon, hydrogenated silicon oxide, and hydrogenated silicon oxynitride. Optionally, the thickness of the first amorphous silicon layer is in a range from about 1 nm to about 2 nm. Optionally, the thickness of the silicon wafer is in a range from about 200 µm to about 500 µm. The material of the second amorphous silicon layer includes, but is not limited to, one or more selected from the group consisting of hydrogenated amorphous silicon, hydrogenated silicon oxide, and hydrogenated silicon oxynitride. Optionally, the thickness of the second amorphous silicon layer 90 is in a range from about 1 nm to about 2 nm. The material of the P-type doped layer includes, but is not limited to, one or more selected from the group consisting of boron-doped amorphous silicon, boron-doped nano silicon (nanocrystalline silicon or microcrystalline silicon), and boron-doped carbon-oxygenated amorphous silicon. Optionally, the thickness of the P-type doped layer is in a range from about 3 nm to about 20 nm. The material of the third transparent conductive layer includes, but is not limited to, one or more selected from the group consisting of indium tin oxide, aluminum zinc oxide, aluminum indium oxide, indium cerium oxide, and indium tungsten oxide. Optionally, the thickness of the third transparent conductive layer is in a range from about 5 nm to about 200 nm.

Similarly, for the photovoltaic device where the anti-reflection layer includes a patterned anti-reflection light-conversion photovoltaic encapsulant film, the electron transport layer can include either a single layer or two layers. For example, in the direction approaching the perovskite active layer, the electron transport layer can include a first electron transport layer and a second electron transport layer. The material of the first electron transport layer includes, but is not limited to, one or more semiconductor metal oxides selected from tin oxide, zinc oxide, and titanium oxide. The material of the second electron transport layer includes, but is not limited to, one or more fullerene derivatives selected from C₆₀, C₇₀, [6,6]-phenyl-C₆₁-butyric acid methyl ester (PC₆₁BM), [6,6]-phenyl-C₇₁-butyric acid methyl ester (PC₇₁BM), or one or more electron transport materials selected from a naphthalene diimide and a perylene diimide-type electron.

It should be understood that, for the photovoltaic device where the anti-reflection layer includes a patterned anti-reflection light-conversion photovoltaic encapsulant film, a hole modification layer can be further disposed between the perovskite active layer and the hole transport layer to further enhance hole transport capability. Further, the material of the hole modification layer includes, but is not limited to, one or more self-assembling materials containing carbazole and phosphonic acid functional groups selected from (4-(3,6-dimethyl-9H-carbazol-9-yl)butyl)phosphonic acid (Me-4PACz), (2-(3,6-dimethoxy-9H-carbazol-9-yl)ethyl)phosphonic acid (MeO-2PACz), and (2-(9H-carbazol-9-yl)ethyl)phosphonic acid (2PACz).

It should be understood that, for the photovoltaic device where the anti-reflection layer includes a patterned anti-reflection light-conversion photovoltaic encapsulant film, the N-type doped layer can be an N-type microcrystalline silicon layer or an N-type amorphous silicon layer, and the P-type doped layer can be a P-type microcrystalline silicon layer or a P-type amorphous silicon layer. The first amorphous silicon layer and the second amorphous silicon layer are each independently an intrinsic hydrogenated amorphous silicon layer.

FIG. 6 is a schematic structural view of a solar cell including two patterned anti-reflection layers according to an embodiment of the present application. Both the second anti-reflection layer (the anti-reflection layer at the light-receiving surface) and the third anti-reflection layer (the anti-reflection layer at the back surface) of the solar cell are the patterned anti-reflection light-conversion photovoltaic encapsulant films.

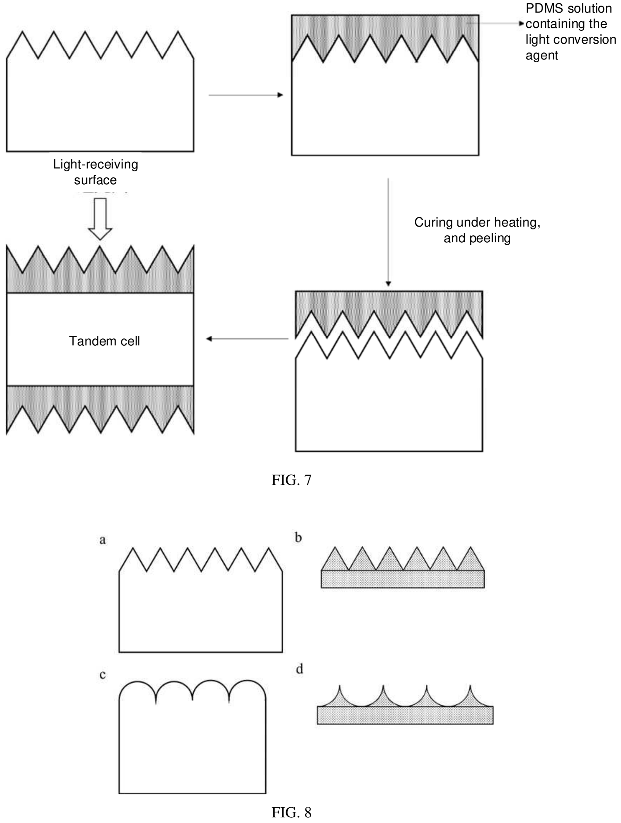

Referring to FIG. 7, the present application further provides a flowchart of a method for preparing the solar cell including two patterned anti-reflection layers as shown in FIG. 6, and a schematic structural view of intermediate structures thereof. The preparation method includes the following steps:
texturing a surface of a silicon wafer to form a patterned textured surface;
preparing an organic silicone resin (PDMS) solution containing a light conversion agent;
applying the PDMS solution containing the light conversion agent to the textured surface, heating to cure the solution, and peeling to obtain a patterned photovoltaic encapsulant film;
attaching the patterned photovoltaic encapsulant film to a surface of a solar cell.

FIG. 8 is schematic structural views of photovoltaic encapsulant films with pyramid-shaped and hemispherical patterns. Optionally, as shown in a and b of FIG. 8, the height of the pyramid-shaped texture is in a range from about 0.01 µm to about 10 µm, with a dimensional error of less than or equal to about 1 µm between the pyramids. As shown in c and d of FIG. 8, the height of the hemispherical texture is in a range from about 0.01 µm to about 10 µm, with a dimensional error of less than or equal to about 1 µm between the hemispheres.

The present application will be further described in conjunction with specific examples below, but the present application is not limited to these examples. It should be understood that the appended claims define the scope of the present application. Under the guidance of the concepts of the present application, those skilled in the art should recognize that modifications to the embodiments of the present application will still fall within the spirit and scope of the claims of the present application.

### I. Examples for synthesizing compounds

### Synthesis Example 1

Synthesis of 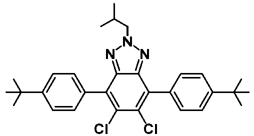 included the following steps:
1 mmol of 4,7-dibromo-5,6-dichloro-2-isobutyl-2H-benzo[d][1,2,3]triazole, 3 mmol (4-(tert-butyl)phenyl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:4 as the eluent to collect the blue fluorescent fraction. The eluates were combined and spin-dried to obtain a white solid.

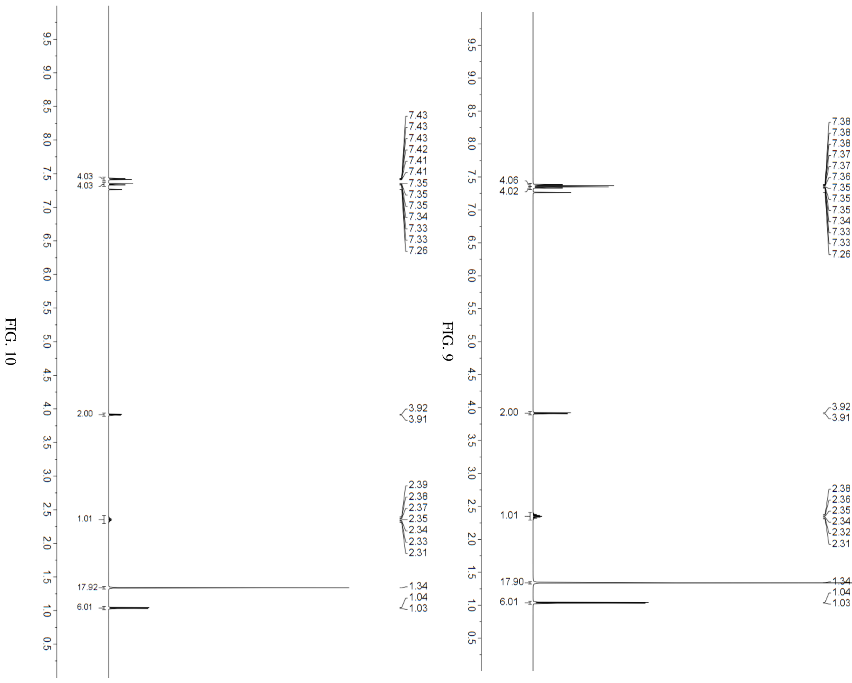

¹H NMR (500 MHz, Chloroform-*d*) δ = 7.45 - 7.39 (m, 4H), 7.37 - 7.31 (m, 4H), 3.91 (d, *J*=7.0, 2H), 2.35 (dh, *J*=13.6, 6.8, 1H), 1.34 (s, 18H), 1.04 (d, *J*=6.8, 6H). The ¹H NMR spectrum is shown in FIG. 9. The mass of the compound was 507.2, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 1.

### Synthesis Example 2

Synthesis of 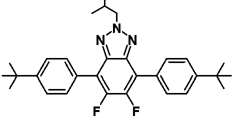 included the following steps:
1 mmol of 4,7-dibromo -5,6-difluoro-2-isobutyl-2H-benzo[d][1,2,3]triazole, 3 mmol (4-(tert-butyl)phenyl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:4 as the eluent to collect the blue fluorescent fraction. The eluates were combined and spin-dried to obtain a white solid.

¹H NMR (500 MHz, Chloroform-*d*) δ = 7.40 - 7.36 (m, 4H), 7.36 - 7.31 (m, 4H), 3.91 (d, *J*=7.0, 2H), 2.34 (dh, *J*=13.7, 6.9, 1H), 1.34 (s, 18H), 1.04 (d, *J*=6.8, 6H). The ¹H NMR spectrum is shown in FIG. 10. The mass of the compound was 476.28, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 2.

### Synthesis Example 3

Synthesis of 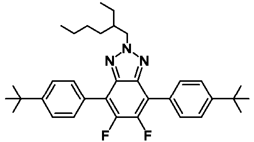 included the following steps:
1 mmol of 4,7-dibromo-5,6-difluoro-2-(2-ethyl-hexyl)-2H-benzo[d] [1, 2,3]triazole, 3 mmol (4-(tert-butyl)phenyl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:8 as the eluent to collect the blue fluorescent fraction. The eluates were combined and spin-dried to obtain a white solid.

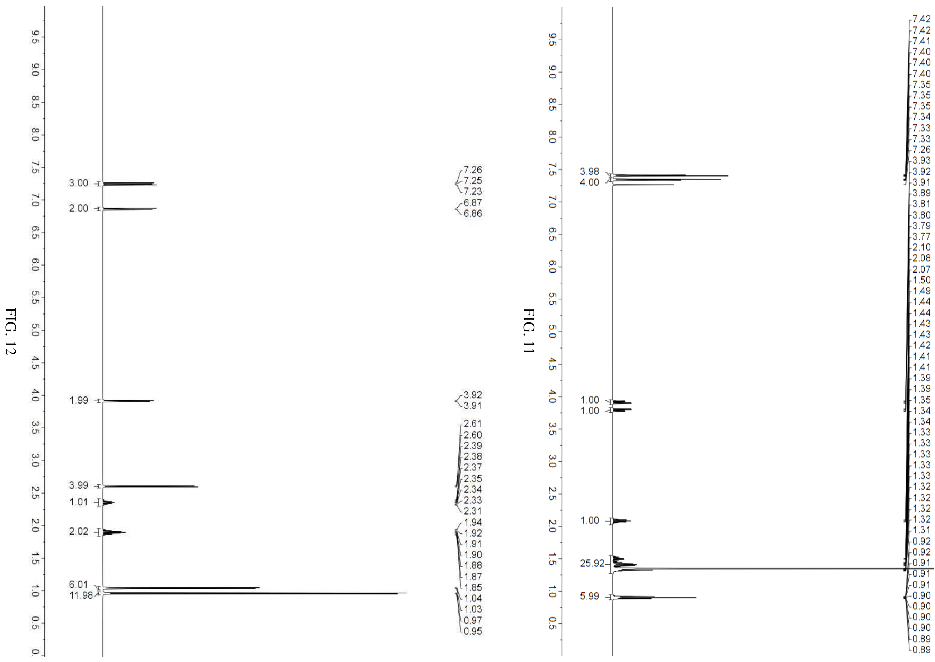

¹H NMR (500 MHz, Chloroform-*d*) δ = 7.43 - 7.38 (m, 4H), 7.37 - 7.31 (m, 4H), 3.91 (dd, *J*=12.4, 7.0, 1H), 3.79 (dd, *J*=12.4, 7.0, 1H), 2.08 (hept, *J*=7.0, 1H), 1.55 - 1.27 (m, 26H), 0.90 (td, *J*=8.0, 1.7, 6H). The ¹H NMR spectrum is shown in FIG. 11. The mass of the compound was 531.3, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 3.

### Synthesis Example 4

Synthesis of 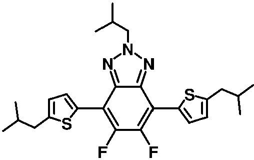 included the following steps:
1 mmol of 4,7-dibromo-5,6-difluoro-2-isobutyl-2H-benzo[d][1,2,3]triazole, 3 mmol (5-isobutylthiophen-2-yl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:6 as the eluent to collect the blue fluorescent fraction. The eluates were combined and spin-dried to obtain a light-yellow solid.

¹H NMR (500 MHz, Chloroform-*d*) δ = 7.28 - 7.21 (m, 2H), 6.86 (d, *J*=7.5, 2H), 3.91 (d, *J*=7.0, 2H), 2.60 (d, *J*=7.0, 4H), 2.35 (dh, *J*=13.6, 6.8, 1H), 1.89 (dh, *J*=13.8, 6.9, 2H), 1.04 (d, *J*=6.8, 6H), 0.96 (d, *J*=6.8, 12H). The ¹H NMR spectrum is shown in FIG. 12. The mass of the compound was 487.2, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 4.

### Synthesis Example 5

Synthesis of 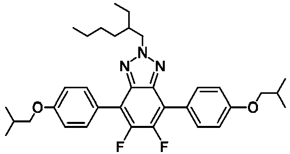 included the following steps:
(1) Synthesis step 1: 1 mmol of 4,7-dibromo-5,6-difluoro-2-(2-ethyl-hexyl)-2H-benzo[d][1,2,3]triazole, 3 mmol (4-isobutoxyphenyl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:3 as the eluent to collect the blue fluorescent fraction. The eluates were combined and spin-dried to obtain a white solid.

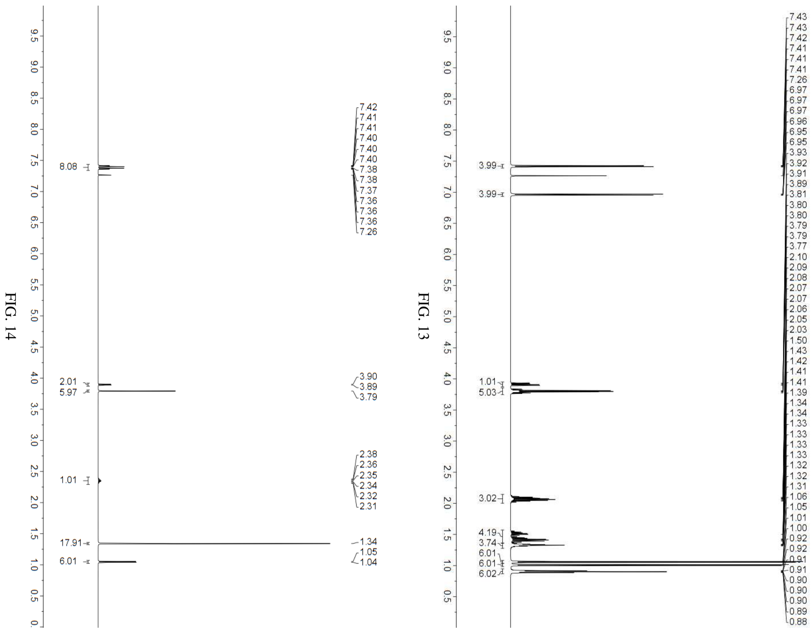

¹H NMR (500 MHz, Chloroform-*d*) δ = 7.45 - 7.39 (m, 4H), 6.99 - 6.93 (m, 4H), 3.91 (dd, *J*=12.4, 7.1, 1H), 3.85 - 3.75 (m, 5H), 2.07 (ddp, *J*=13.7, 10.3, 6.9, 3H), 1.57 - 1.36 (m, 4H), 1.36 - 1.28 (m, 4H), 1.06 (d, *J*=6.8, 6H), 1.01 (d, *J*=6.7, 6H), 0.90 (td, *J*=8.0, 1.2, 6H). The ¹H NMR spectrum is shown in FIG. 13. The mass of the compound was 563.3, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 5.

### Synthesis Example 6

Synthesis of 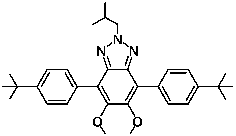 included the following steps:
1 mmol of 4,7-dibromo-2-isobutyl-5,6-dimethoxy-2H-benzo[d][1,2,3]triazole, 3 mmol (4-(tert-butyl)phenyl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:3 as the eluent to collect the blue fluorescent fraction. The eluates were combined and spin-dried to obtain a white solid.

¹H NMR (500 MHz, Chloroform-*d*) δ = 7.44 - 7.34 (m, 8H), 3.90 (d, *J*=7.0, 2H), 3.79 (s, 6H), 2.34 (dh, *J*=13.7, 6.9, 1H), 1.34 (s, 18H), 1.05 (d, *J*=6.8, 6H). The ¹H NMR spectrum is shown in FIG. 14. The mass of the compound was 499.3, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 6.

### Synthesis Example 7

Synthesis of 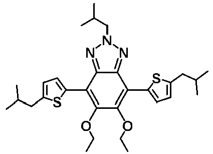 included the following steps:
1 mmol of 4,7-dibromo-5,6-diethoxy-2-isobutyl-2H-benzo[d][1,2,3]triazole, 3 mmol (5-isobutylthiophen-2-yl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:4 as the eluent to collect the blue fluorescent fraction. The eluates were combined and spin-dried to obtain a light-yellow solid.

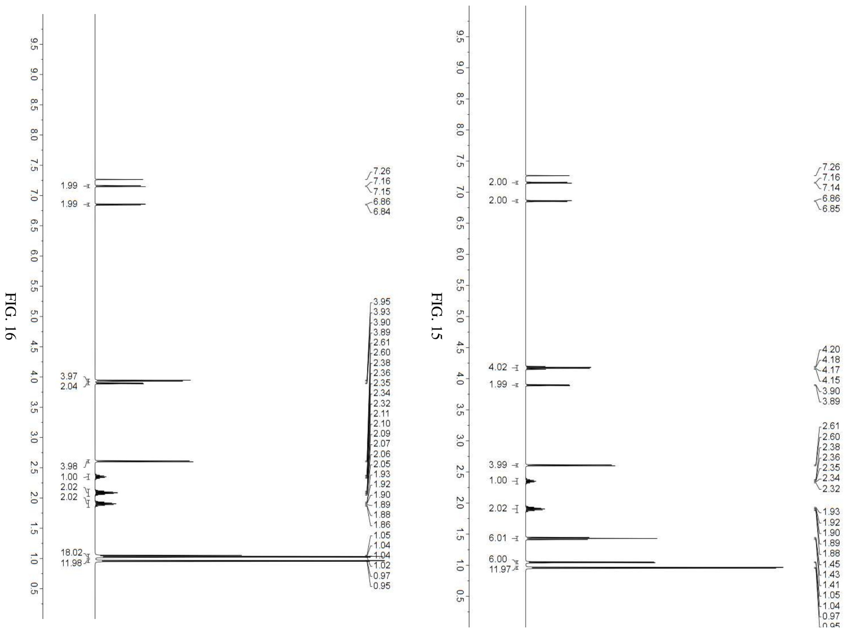

¹H NMR (500 MHz, Chloroform-*d*) δ = 7.15 (d, *J*=7.5, 2H), 6.86 (d, *J*=7.5, 2H), 4.17 (q, *J*=8.0, 4H), 3.90 (d, *J*=7.0, 2H), 2.61 (d, *J*=7.0, 4H), 2.35 (dp, *J*=13.6, 6.9, 1H), 1.90 (dp, *J*=13.6, 6.9, 2H), 1.43 (t, *J*=8.0, 6H), 1.05 (d, *J*=6.8, 6H), 0.96 (d, *J*=6.8, 12H). The ¹H NMR spectrum is shown in FIG. 15. The mass of the compound was 539.3, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 7.

### Synthesis Example 8

Synthesis of 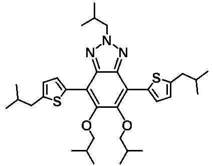 included the following steps:
1 mmol of 4,7-dibromo-3,6-diisobutoxy-2-isobutyl-2H-benzo[d][1,2,3]triazole, 3 mmol (5-isobutylthiophen-2-yl)boronic acid, and 3 mmol potassium carbonate were weighed and introduced to a 50 mL two-neck flask. The two-neck flask was connected with a vacuum hose, evacuated and filled with nitrogen. The evacuation and filling were repeated three times to ensure an inert atmosphere in the two-neck flask. Then 10 mL of 1,4-dioxane and 10 mL of deionized water were added and uniformly mixed under stirring. The mixture was reacted at 95 °C for 24 hours. After that, 20 mL of deionized water was added to quench the reaction. The reaction solution was extracted with dichloromethane three times. The organic layers were combined and spin-dried to obtain a crude product. The crude product was purified using a 200-300 mesh silica gel chromatography column, with dichloromethane and petroleum ether in a volume ratio of 1:4 as the eluent to collect the blue fluorescent fraction. The eluates were combined and spin-dried to obtain a light-yellow solid.

¹H NMR (500 MHz, Chloroform-*d*) δ = 7.15 (d, *J*=7.5, 2H), 6.85 (d, *J*=7.5, 2H), 3.94 (d, *J*=7.0, 4H), 3.90 (d, *J*=7.0, 2H), 2.61 (d, *J*=7.0, 4H), 2.35 (hept, *J*=6.9, 1H), 2.08 (dh, *J*=13.7, 6.9, 2H), 1.90 (dp, *J*=13.6, 6.9, 2H), 1.04 (dd, *J*=8.6, 6.8, 18H), 0.96 (d, *J*=6.8, 12H). The ¹H NMR spectrum is shown in FIG. 16. The mass of the compound was 595.3, determined by Matrix Assisted Laser Desorption/Ionization Time-of-Flight (MALDI-TOF).

Based on the ¹H NMR and mass spectrometry results, it was confirmed that the target compound was successfully synthesized in Synthesis Example 8.

### Device Example 1

This example provides a solar cell as shown in FIG. 3, a photovoltaic device as shown in FIG. 4, and their preparation methods, as follows:
1. Preparation of the solar cell as shown in FIG. 3, with the following steps:
   A silicon wafer with a thickness of 280 µm was cleaned and textured. Subsequently, a first amorphous silicon layer with a thickness of 2 nm and a second amorphous silicon layer with a thickness of 2 nm were formed using PECVD. An N-type doped layer with a thickness of 5 nm and a P-type amorphous silicon layer with a thickness of 10 nm were then formed. Next, by using magnetron sputtering, a second transparent conductive layer with a thickness of 20 nm was formed on the N-type doped layer, and a third transparent conductive layer with a thickness of 40 nm was formed on the P-type doped layer. A second finger electrode layer was then printed on the third transparent conductive layer. A hole transport layer with a thickness of 10 nm was then formed on the second transparent conductive layer, followed by depositing a hole modification layer with a thickness of 1 nm and then depositing a perovskite active layer with a thickness of 0.8 µm using a solution method. Next, a second electron transport layer with a thickness of 20 nm was deposited by using thermal evaporation, and a first electron transport layer with a thickness of 20 nm was deposited by using atomic layer deposition. Then, by using magnetron sputtering, a first transparent conductive layer with a thickness of 80 nm was formed on the first electron transport layer and a MgFₓ layer with a thickness of 80 nm was formed on the surface of the first transparent conductive layer, followed by printing a first finger electrode layer to obtain the solar cell.
2. Preparation of the photovoltaic device as shown in FIG. 4, with the following steps:
   (1) Ethylene-vinyl acetate, the benzotriazole compound from Synthesis Example 1, tert-butylperoxy 2-ethylhexyl carbonate (TBEC), silane coupling agent KH570, and antioxidant 152 were mixed and then dried to form a film, thereby obtaining a photovoltaic encapsulant film, which can be referred to as a light-converting encapsulant film.
      By mass percentage, the light-converting encapsulant film includes 99% ethylene-vinyl acetate, 0.2% of the benzotriazole compound in Synthesis Example 1, 0.3% TBEC, 0.3% silane coupling agent KH570, and 0.2% antioxidant 152.
   (2) A front glass plate with a desired size was selected. The above light-converting encapsulant film was cut to match the size and stacked on the front glass plate. Then, a string of electrically connected cells, a second encapsulant film, and a back glass plate were sequentially stacked on the light-converting encapsulant film. The stack was laminated in a laminator to obtain a laminated module. Subsequently, a junction box and a metal frame were installed to obtain the photovoltaic device as shown in FIG. 4.

### Device Example 2

This example provides a solar cell as shown in FIG. 3, a photovoltaic device as shown in FIG. 4, and their preparation methods, as follows:
1. Preparation of the solar cell as shown in FIG. 3, with the same steps as those in Device Example 1.
2. Preparation of the photovoltaic device as shown in FIG. 4, the steps were substantially the same as those in Device Example 1, except that:
   By mass percentage, the light-converting encapsulant film includes 99.5% poly(ethylene-co-octene) elastomer and 0.5% of the benzotriazole compound from Synthesis Example 2.

### Device Example 3

This example provides a solar cell as shown in FIG. 3, a photovoltaic device as shown in FIG. 4, and their preparation methods, as follows:
1. Preparation of the solar cell as shown in FIG. 3, with the same steps as those in Device Example 1.
2. Preparation of the photovoltaic device as shown in FIG. 4, the steps were substantially the same as those in Device Example 1, except that:
   By mass percentage, the light-converting encapsulant film includes 99% organic silicone resin DC184 from Dow, 0.5% of the benzotriazole compound from Synthesis Example 3, and 0.5% antioxidant 152.

In Device Examples 4 to 8 and Device Comparative Examples 1 to 3, different solar cells and photovoltaic devices were prepared according to the method described in Device Example 1 and the contents shown in Table 1, with the type of light conversion agent changed. The light conversion agents used in Device Comparative Examples 1 to 3 were purchased from Zhengzhou Alfa Chemical Co., Ltd.

The solar cells and photovoltaic devices from Device Examples 1 to 8 and Device Comparative Examples 1 to 3 were subjected to performance tests. The test methods were as follows:
(1) Light conversion efficiency: The ratio of the number of photons emitted as secondary fluorescent radiation to the number of photons absorbed from the primary excitation radiation per unit time. The results are shown in Table 1.
(2) Photovoltaic efficiency: The efficiency of the solar cells was measured under AM1.5G at 25 °C using an IV test system. The results are shown in Table 1.
(3) T80 test: Using an AM1.5G simulated xenon lamp spectrum, the solar cells were continuously tracked at the maximum power point, and the time taken for the cell efficiency to degrade to 80% was recorded. The results are shown in Table 1.

**Table 1**

| Device | Small molecule light conversion agent | Maximum absorption wavelength (nm) | Maximum emission wavelength (nm) | Light conversion efficiency (%) | Photovoltaic efficiency (%) | T80 (h) |
|---|---|---|---|---|---|---|
| Device Example 1 | 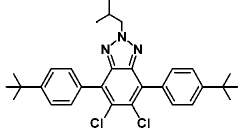 | 355 | 415 | 88 | 30.8 | 5524 |
| Device Example 2 | 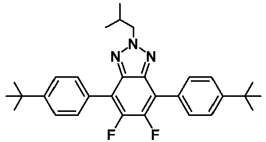 | 360 | 420 | 95 | 31.1 | 3321 |
| Device Example 3 | 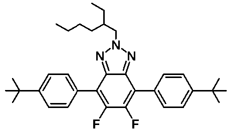 | 358 | 417 | 94 | 29.8 | 3513 |
| Device Example 4 | 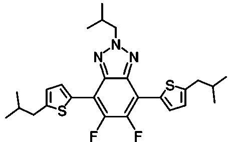 | 380 | 440 | 90 | 28.8 | 5035 |
| Device Example 5 | 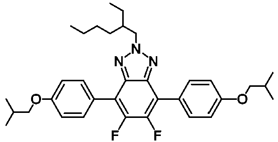 | 375 | 430 | 97 | 31.5 | 7271 |
| Device Example 6 | 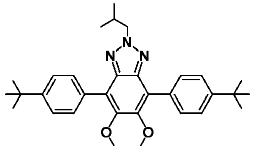 | 375 | 425 | 88 | 30.6 | 6720 |
| Device Example 7 | 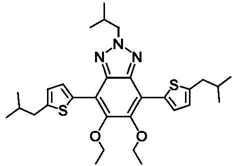 | 385 | 445 | 95 | 29.1 | 4602 |
| Device Example 8 | 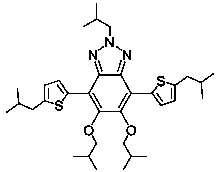 | 400 | 470 | 90 | 28.7 | 3960 |
| Device Comparative Example 1 | 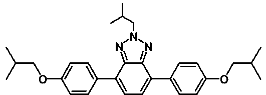 | 410 | 485 | 72 | 26.7 | 2960 |
| Device Comparative Example 2 | 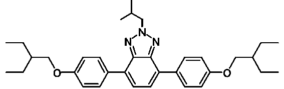 | 410 | 485 | 72 | 26.5 | 2870 |
| Device Comparative Example 3 | 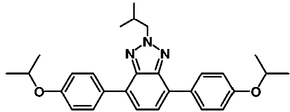 | 405 | 480 | 70 | 26.3 | 1960 |

It can be seen from Table 1 that, compared to Device Comparative Examples 1 to 3, the photovoltaic devices of Device Examples 1 to 8, using the benzotriazole organic compounds from Synthesis Examples 1 to 8 of the present application as light conversion agents, respectively, exhibit improved light conversion efficiency, photovoltaic efficiency, and service life.

### Device Example 9

This example provides a solar cell as shown in FIG. 6 and a preparation method thereof. The steps were as follows:
(1) A silicon wafer with a thickness of 280 µm was cleaned and textured. Subsequently, a first amorphous silicon layer with a thickness of 2 nm and a second amorphous silicon layer with a thickness of 2 nm were formed using PECVD. An N-type doped layer with a thickness of 5 nm and a P-type amorphous silicon layer with a thickness of 10 nm were then formed. Next, by magnetron sputtering, a second transparent conductive layer with a thickness of 20 nm was formed on the N-type doped layer, and a third transparent conductive layer with a thickness of 40 nm was formed on the P-type doped layer. A second finger electrode layer was then printed on the third transparent conductive layer. A hole transport layer with a thickness of 10 nm was then formed on the second transparent conductive layer, followed by depositing a hole modification layer with a thickness of 1 nm and then depositing a perovskite active layer with a thickness of 0.8 µm using a solution method. Next, a second electron transport layer with a thickness of 20 nm was deposited by using thermal evaporation, and a first electron transport layer with a thickness of 20 nm was deposited by using atomic layer deposition. Then, a first transparent conductive layer with a thickness of 80 nm was formed on the first electron transport layer by using magnetron sputtering.
(2) 1 g of hydrogen-containing silicone (the crosslinking agent component of silicone DC184 ) was weighed, followed by adding 11 mg of the benzotriazole compound from Synthesis Example 5 and then adding 10 g of vinyl-terminated PDMS. The mixture was stirred uniformly and degassed under vacuum to form a transparent solution. 10 mL of the transparent solution was applied to surfaces of a textured silicon wafer. The textured silicon wafer was 10 cm × 10 cm sized with a pyramid height of 0.5 µm. The transparent solution was spin-coated at a speed of 500 rpm for 60 seconds using a spin coater, then the silicon wafer was heated to 120 °C and held for 15 minutes to complete curing. After cooling to room temperature, pyramid-structured photovoltaic encapsulant films were peeled off from the surfaces of the silicon wafer.
(3) The photovoltaic encapsulant films was used as the second and third anti-reflection layers, respectively, with their smooth sides attached to the surfaces of the first and third transparent conductive layers.
(4) The first finger electrode layer was printed to obtain the solar cell as shown in FIG. 6.

### Device Comparative Example 4

Device Comparative Example 4 was substantially the same as Device Example 9, except that the second and third anti-reflection layers each consisted of 99.9% EVA and 0.1% UV-329 by mass percentage.

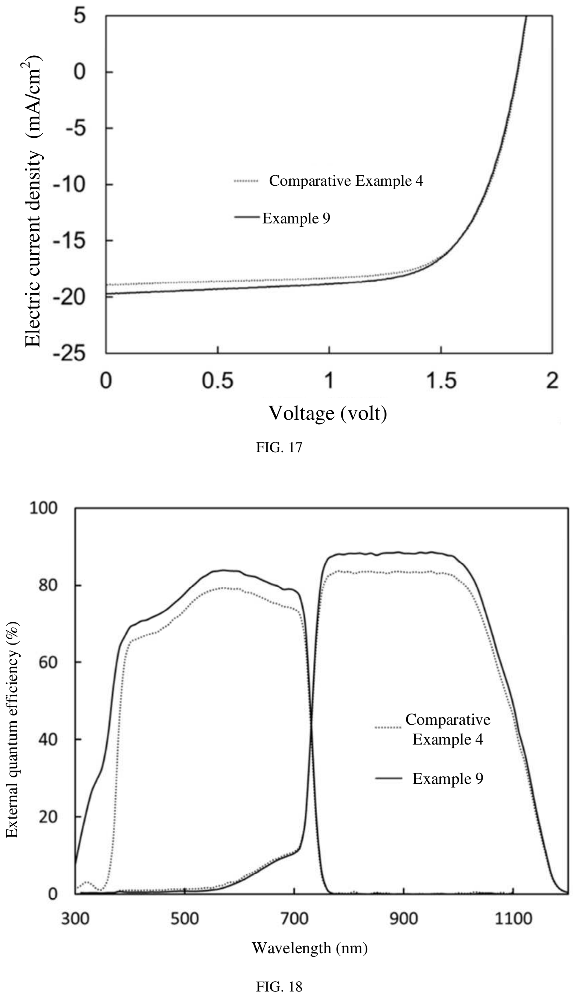

The solar cells from Device Example 9 and Device Comparative Example 4 were subjected to the current-voltage (IV) and external quantum efficiency (EQE) performance testing, and the results are shown in FIGs. 17 and 18. FIG. 17 shows the I-V curves of Device Example 9 and Device Comparative Example 4. It can be seen from FIG. 17 that Device Example 9 exhibits a significant improvement in short-circuit current density, thereby enhancing cell efficiency. FIG. 18 shows the EQE curves of Device Example 9 and Device Comparative Example 4. It can be seen from FIG. 18 that compared to Device Comparative Example 4, Device Example 9 demonstrates a significant improvement in the short-wavelength region and an overall enhanced EQE response due to the presence of the anti-reflection layers, leading to an increase in short-circuit current density.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present application, and the descriptions are relatively specific and detailed, but they should not be construed as limiting the scope of the present application. It should be understood by those of ordinary skill in the art that various modifications and improvements can be made without departing from the concept of the present application, and all fall within the protection scope of the present application. Therefore, the patent protection of the present application shall be defined by the appended claims.

## Claims

1. A benzotriazole organic compound having a general structural formula (1): 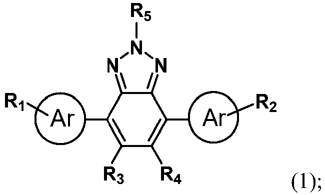
wherein each Ar independently includes substituted or unsubstituted aryl, or substituted or unsubstituted heteroaryl;
R₁ and R₂ are each independently selected from substituted or unsubstituted alkyl, or substituted or unsubstituted alkoxy;
R₃ and R₄ are each independently selected from halogen, substituted or unsubstituted alkoxy, or substituted or unsubstituted alkylthio; and
R₅ is selected from substituted or unsubstituted alkyl.

2. The benzotriazole organic compound according to claim 1, wherein each Ar independently includes substituted or unsubstituted aryl having 6 to 30 ring atoms, or substituted or unsubstituted heteroaryl having 5 to 30 ring atoms.

3. The benzotriazole organic compound according to claim 2, wherein each Ar is independently selected from substituted or unsubstituted six-membered aryl, substituted or unsubstituted six-membered heteroaryl, or substituted or unsubstituted five-membered heteroaryl.

4. The benzotriazole organic compound according to claim 3, wherein each Ar is independently selected from one of following groups: 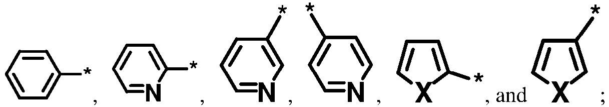 wherein * represents a linking site, and X represents O, S, or Se.

5. The benzotriazole organic compound according to any one of claims 1 to 4, wherein R₁ and R₂ are each independently selected from substituted or unsubstituted C₁ to C₂₀ alkyl, or substituted or unsubstituted C₁ to C₂₀ alkoxy.

6. The benzotriazole organic compound according to claim 5, wherein R₁ and R₂ are each independently selected from one of following groups: 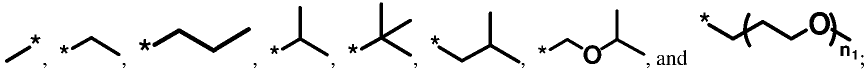 wherein * represents a linking site, and n₁≥2.

7. The benzotriazole organic compound according to any one of claims 1 to 6, wherein R₃ and R₄ are each independently selected from halogen, substituted or unsubstituted C₁ to C₂₀ alkoxy, or substituted or unsubstituted C₁ to C₂₀ alkylthio.

8. The benzotriazole organic compound according to claim 7, wherein R₃ and R₄ are each independently selected from one of following groups: 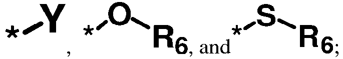 wherein * represents a linking site, Y represents F, Cl, Br, or I, and each R₆ is independently selected from substituted or unsubstituted C₁ to C₂₀ linear alkyl, or substituted or unsubstituted C₃ to C₂₀ branched alkyl.

9. The benzotriazole organic compound according to any one of claims 1 to 8, wherein R₅ is selected from substituted or unsubstituted C₁ to C₂₀ alkyl.

10. The benzotriazole organic compound according to claim 9, wherein R₅ is selected from one of following groups: 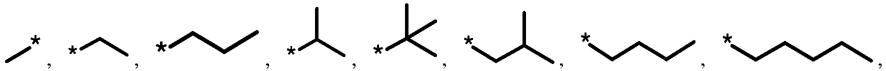 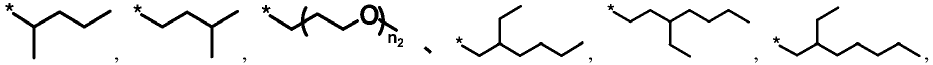 and 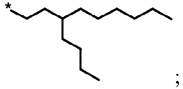 wherein * represents a linking site, and n₂≥2.

11. The benzotriazole organic compound according to any one of claims 1 to 10, having any one of following structures:

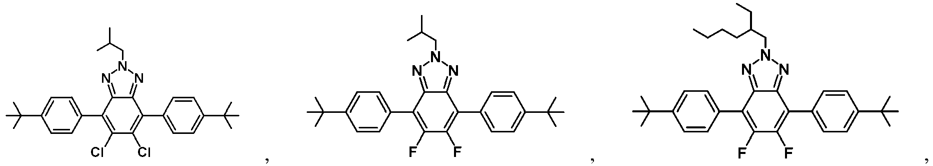

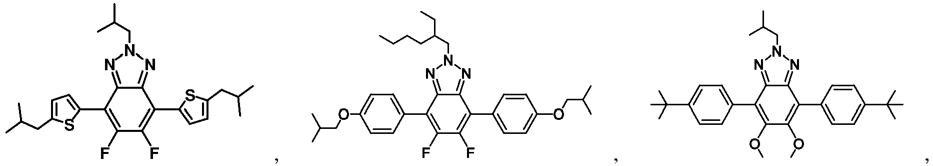

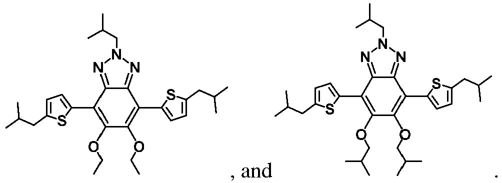

12. A method for preparing the benzotriazole organic compound according to any one of claims 1 to 11, comprising following steps:
mixing and reacting compound (a), compound (b), compound (c), and an alkali in a solvent to prepare the benzotriazole organic compound; 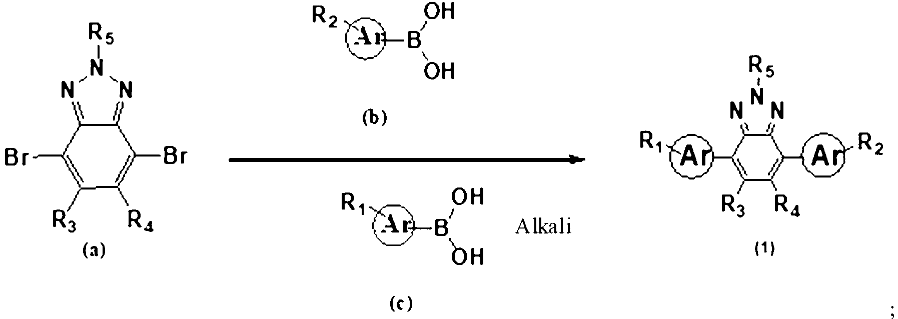
wherein definitions of R₁, R₂, R₃, R₄, and R₅ are same as those in any one of claims 1 to 11.

13. A light conversion agent comprising the benzotriazole organic compound according to any one of claims 1 to 11.

14. The light conversion agent according to claim 13, having a light absorption in a wavelength range from about 280 nm to about 400 nm.

15. The light conversion agent according to claim 13 or 14, having a maximum absorption peak at about 320 nm to about 380 nm.

16. The light conversion agent according to any one of claims 13 to 15, having a photoluminescence in a range from about 400 nm to about 600 nm.

17. The light conversion agent according to any one of claims 13 to 16, having a maximum emission peak at about 450 nm to about 550 nm.

18. A photovoltaic encapsulant film, comprising the benzotriazole organic compound according to any one of claims 1 to 11, or the light conversion agent according to any one of claims 13 to 17.

19. The photovoltaic encapsulant film according to claim 18, further comprising an optically transparent polymer material.

20. The photovoltaic encapsulant film according to claim 19, by mass percentage, comprising about 0.01% to about 5% of the benzotriazole organic compound according to any one of claims 1 to 11 or the light conversion agent according to any one of claims 13 to 17, and about 95% to about 99.99% of the polymer material.

21. The photovoltaic encapsulant film according to claim 19 or 20, wherein the polymer material is one or more selected from the group consisting of a polyolefin elastomer, an ethylene-vinyl acetate copolymer, a polyvinyl butyral, and an organic silicone resin material.

22. The photovoltaic encapsulant film according to claim 21, wherein the organic silicone resin material includes a mixture of a hydrogen-containing silicone and a vinyl-terminated polydimethylsiloxane in a mass ratio of substantially 1:(2 to 15).

23. A photovoltaic device comprising a solar cell and the photovoltaic encapsulant film according to any one of claims 18 to 22, wherein the photovoltaic encapsulant film is disposed on at least one surface of the solar cell.

24. The photovoltaic device according to claim 23, wherein the solar cell includes a crystalline silicon cell, a perovskite cell, a tandem cell including the perovskite cell and the crystalline silicon cell, or any combination thereof.

25. The photovoltaic device according to claim 24, wherein the solar cell is the tandem cell including the perovskite cell and the crystalline silicon cell, and the solar cell includes a first transparent conductive layer, an electron transport layer, a perovskite active layer, a hole transport layer, a second transparent conductive layer, an N-type doped layer, a first amorphous silicon layer, a silicon wafer, a second amorphous silicon layer, a P-type doped layer, and a third transparent conductive layer stacked in sequence, and further includes a first finger electrode layer and a second finger electrode layer,
wherein the first finger electrode layer includes a plurality of first finger electrodes spaced apart from each other, and each first finger electrode is electrically connected to the first transparent conductive layer; and
the second finger electrode layer includes a plurality of second finger electrodes spaced apart from each other, and each second finger electrode is electrically connected to the third transparent conductive layer.

26. The photovoltaic device according to claim 25, further comprising a light-receiving surface plate and a back surface plate, the light-receiving surface plate is stacked on a surface of the first transparent conductive layer away from the perovskite active layer, the back surface plate is stacked on a surface of the third transparent conductive layer away from the perovskite active layer, and surfaces of the solar cell are coated with the photovoltaic encapsulant films.

27. The photovoltaic device according to claim 25 or 26, wherein the solar cell further includes a first anti-reflection layer, the first anti-reflection layer is stacked on the surface of the first transparent conductive layer away from the perovskite active layer, and the first finger electrodes extend through the first anti-reflection layer and are electrically connected to the first transparent conductive layer;
a material of the first anti-reflection layer includes magnesium fluoride, lithium fluoride, silicon nitride, silicon oxide, silicon oxynitride, or any combination thereof.

28. The photovoltaic device according to any one of claims 25 to 27, wherein the solar cell further includes a second anti-reflection layer, the second anti-reflection layer is stacked on the surface of the first transparent conductive layer away from the perovskite active layer, the first finger electrodes extend through the second anti-reflection layer and are electrically connected to the first transparent conductive layer;
the second anti-reflection layer includes a photovoltaic encapsulant film according to claim 22, and the photovoltaic encapsulant film is patterned.

29. The photovoltaic device according to any one of claims 25 to 28, wherein the solar cell further includes a third anti-reflection layer, the third anti-reflection layer is stacked on the surface of the third transparent conductive layer away from the perovskite active layer, the second finger electrodes extend through the third anti-reflection layer and are electrically connected to the third transparent conductive layer;
the third anti-reflection layer includes a photovoltaic encapsulant film according to claim 22, and the photovoltaic encapsulant film is patterned.
